# EUROPEAN PATENT APPLICATION

(11) **EP 4 156 717 A1**
(43) Date of publication of application: **29.03.2023**
(21) Application number: 21198947.0
(22) Date of filing: 24.09.2021
(51) Int. Cl.: H04R 25/00, A61B 5/00

(54) **METHOD AND COMPUTER PROGRAM FOR EARLY DETECTION OF A HEALTH ISSUE, CONTROLLER FOR A HEARING DEVICE, HEARING DEVICE, AND HEARING SYSTEM COMPRISING A HEARING DEVICE**

(71) Applicant: Sonova AG, 8712 Stäfa (CH)
(72) Inventor: Breitenmoser, Andreas, 8004 Zürich (CH); Thielen, Anne, 8712 Stäfa (CH)
(74) Representative: Qip Patentanwälte Dr. Kuehn & Partner mbB

(57) **Abstract**

A method for early detection of a health issue of a user wearing a hearing device (12) is provided. The hearing device (12) comprises at least one sound detector (20) for generating an audio signal, at least one user detector for generating a user detection signal, a processing unit (23) for modifying the audio signal, and at least one sound output component (24) for outputting the modified audio signal. The method comprises: receiving (S2) the audio signal and/or the user detection signal for a predetermined time interval; determining (S4) a current activity pattern of the user by evaluating the audio signal and/or the user detection signal received over the predetermined time interval; evaluating (S6) the current activity pattern; determining (S8) whether there is an upcoming health issue of the user depending on the evaluation; and providing (S10), if it is determined that there is an upcoming health issue of the user, an output signal indicative of the health condition.

## Description

### FIELD OF THE INVENTION

The invention relates to a method and a computer program for early detection of a health issue, a hearing device, and a hearing system comprising a hearing device.

### BACKGROUND OF THE INVENTION

A treatment in the early stages of a health issue can sustainably influence the course of the corresponding sickness in a positive direction. For example, as elderly people's health state can change drastically, a timely detection of arising sicknesses may be essential.

Hearing devices are generally small and complex devices. Hearing devices can include a processor, microphone, an integrated loudspeaker as a sound output device, memory, housing, and other electronical and mechanical components, e.g. at least one sensor for detecting an activity of a user wearing the hearing device. Some example hearing devices are Behind-The-Ear (BTE), Receiver-In-Canal (RIC), In-The-Ear (ITE), Completely-In-Canal (CIC), and Invisible-In-The-Canal (IIC) devices. The user may prefer one of these hearing devices compared to another device based on hearing loss, aesthetic preferences, lifestyle needs, and budget.

A hearing system comprises such a hearing device and a user device of a user of the hearing device. The user device may be a smartphone, a tablet, or any other mobile device. The user device is connected to the hearing device, e.g. wired or wireless, e.g. via Bluetooth. The hearing device and the connected user device may transfer data from one to the other. For example, a computing task, which may consume large computing resources and/or storage space, may be outsourced from the hearing device to the connected user device. Further, if the hearing device needs data from the internet the data may be provided to the hearing device via the connected user device.

It is known that such a hearing device and/or hearing system may be configured in accordance with the preferences and/or needs of the user of the hearing device. For example, one or more sound programs considering the preferences and/or needs of a user may run on the hearing device, wherein the sound output of the hearing device may be modified in accordance with the sound program. The sound program may be changed in accordance with the acoustic environment of the user. For example, a first sound program may be used if the user is in a conversation and a second sound program, which is different from the first sound program, may be used if the user listens to music. For that purpose, the hearing device and/or the user device may comprise a classifier for classifying the acoustic environment of the user.

### DESCRIPTION OF THE INVENTION

It is an objective of the invention to provide a method and a computer program for early detection of a health issue of a user wearing a hearing device, which positively contribute to the health of a user wearing the hearing device. It is another object to allow such a detection with high accuracy, in particular to minimize the detection of false positives. Further, it is an objective of the invention to provide a hearing device and/or hearing system carrying out the method.

This objective is achieved by the subject-matter of the independent claims. Further exemplary embodiments are evident from the dependent claims and the following description.

An aspect of the invention relates to a method for early detection of a health issue of a user wearing a hearing device. The hearing device is configured to be worn at an ear of the user. The hearing device comprises at least one sound detector, e.g. a microphone, for generating an audio signal indicative of a sound detected in an environment of the user, at least one user detector for generating a user detection signal indicative of a property of the user and/or an activity performed by the user, a processing unit for modifying the audio signal, and at least one sound output component for outputting the modified audio signal. The method comprises: receiving the audio signal and/or the user detection signal for a predetermined time interval, in particular as an input signal; determining a current activity pattern of the user by evaluating the audio signal and/or the user detection signal received over the predetermined time interval; evaluating the current activity pattern; determining whether there is an upcoming health issue of the user depending on the evaluation; and providing, if it is determined that there is an upcoming health issue of the user, an output signal indicative of the health issue. The output signal may be a predetermined early detection signal.

The determination of the current activity pattern may relate to recognizing, reconstructing, and/or generating the current activity patterns, e.g. an activity diary. The evaluation of the current activity patterns may relate to analysing the current activity pattern and/or, in case, comparing it to another activity pattern, for example a healthy activity pattern and/or an unhealthy activity pattern.

Many health issues cause a change of the activity, in other words the behaviour, of a person at an early stage of the corresponding illness. Especially cognitive decline or other brain illnesses, such as dementia or Alzheimer's disease, can be detected early through the observation of a daily activity pattern. Therefore, determining and evaluating the current activity pattern and determining whether there is an upcoming health issue of the user depending on the evaluation may contribute to an early detection of the health issue, i.e. the illness, and as such enable to treat the health issue at a very early stage. This increases the chances of successfully treating the health issue and/or healing of the user.

According to an embodiment of the invention, the audio signal may be representative for the acoustic environment of the sound input component and as such of the hearing device and the user.

According to an embodiment of the invention, the audio signal may be modified by the processing unit in accordance with the needs and/or preferences of the user. For example, the audio signal may be amplified in sum or in certain frequency bands, in which the hearing capability of the user is limited. Additionally or alternatively, noise may be removed from the audio signal by the processing unit.

According to an embodiment of the invention, the user detector may include any type(s) of physical sensor(s), like a motion sensor, e.g. an accelerometer or a gyroscope, a magnetic field sensor, a touch-sensitive sensor, e.g. capacitive sensor, an optical sensor, or a temperature sensor etc., integrated in the hearing device or possibly also in a connected user device such as a smartphone or a smartwatch. The motion sensor may enable to monitor an activity of the user. The touch-sensitive sensor may be used as input means of the hearing device, in particular as a user interface. The temperature sensor may be used to determine the body temperature of the user. The optical sensor may be used to measure the pulse, blood pressure, and/or blood oxygen saturation of the user.

According to an embodiment of the invention, the sound output component may be a speaker of the hearing device.

According to an embodiment of the invention, the predetermined time interval during which the audio signal is monitored for determining the current activity pattern may be for example 12h, 24h, 20 days, one week, or one month.

According to an embodiment of the invention, an activity pattern, e.g. the current activity pattern, may be characterized by parameter values of a set of parameters. The parameters may be at least one of the group of steps count of the user; time the user spent in different activity levels; level of body movement and orientation; occurrence of head gestures like nodding or shaking; single or double tap, tap and hold, and/or swipe up/down of an input mean of the hearing device or the connected user device by the user; time the user spent in different social situations; additional interactions, e.g. configuration interactions, such as e.g. need for help menu in an App, or adjustments in the field; parameters representative for the need for making calls and/or patterns of callers; the frequency and/or the duration of listening to music, types of the music style; on/off-and/or charging times of the hearing device.

According to an embodiment of the invention, the input signal from the sound detector and/or the user detector may be representative for at least some of the parameter values. Further parameter values may be derived from signals of at least one of a controller, processor, transceiver, classifier, and power management of the hearing device.

According to an embodiment of the invention, the predetermined early detection signal may be provided to the user and/or a medical professional of the user.

The term "early detection" is used in this application as a fixed medical term as it is used by medical professionals. In particular, the term "early" is not used as a relative term in this context as it may be used in common language. Alternatively, the term "early detection" may be used synonymous for "detection". So, the above method may be also referred to as method for detection of a health issue of a user wearing a hearing device.

The method may be a computer-implemented method, which may be performed automatically by a hearing device and/or a hearing system, comprising the hearing device. The hearing system may, for instance, comprise one or two hearing devices used by the same user. One or both of the hearing devices may be worn on or in an ear of the user. The hearing device may be a hearing aid, which may be adapted for compensating a hearing loss of the user. Also, the hearing device may be a cochlear implant. The hearing system may comprise at least one connected user device, such as a smartphone, smartwatch, other devices carried by the user or a personal computer of the user etc. The user device may be connected to a cloud and/or the internet. Some of the steps of the method described here and further below may be executed on the hearing device, the user device or in the cloud, or any combination thereof.

According to an embodiment of the invention, the current activity pattern may be evaluated by a neuronal network, which has been trained in advance to differentiate an activity pattern of a healthy person from an activity pattern of an unhealthy person. The neuronal network may be a deep neuronal network (DNN). The neuronal network may have been trained with datasets of healthy and/or unhealthy people. In particular, the neuronal network may have been trained with datasets of people having a brain disease, e.g. dementia, Alzheimer's disease, and/or cognitive decline.

According to an embodiment of the invention, the current activity pattern may be evaluated by comparing the current activity pattern with at least one previous activity pattern of the user and it is determined that there is an upcoming health issue of the user if the current activity pattern differs from the previous activity pattern in a predetermined way. The comparison may be carried out by a neuronal network or by a "classical" algorithm. The classical algorithm may comprise, for instance, a rule-based algorithm and/or a tree-based algorithm and/or a probabilistic algorithm. The neuronal network may have been trained with datasets of healthy and/or unhealthy people. In particular, the neuronal network may have been trained with datasets of people having a brain disease, e.g. dementia, Alzheimer's disease, and/or cognitive decline. The classical algorithm may be configured to determine a difference value being representative for a difference between the current activity pattern and the previous activity pattern, to compare the difference value with a predetermined threshold, and to determine that there is an upcoming health issue of the user, if the difference value exceeds the predetermined threshold. For instance, the difference value may be a distance measure or a similarity measure. The previous activity patterns may be sampled in advance over weeks, months, or years. The parameter values characterizing the previous may be stored in the hearing device or in the connected user device.

According to an embodiment of the invention, the previous activity pattern may represent an average of previously determined and stored activity patterns of the user. The current activity pattern may be compared with the previous activity patterns by calculating an average value for each parameter value of the previous activity patterns, by determining an average activity pattern from these average values, and by comparing the current activity pattern with the average activity pattern. Alternatively, the parameter values of the current activity pattern may be compared to the corresponding average values directly without determining the average activity pattern.

According to an embodiment of the invention, after evaluating the current activity pattern, the previous activity pattern may be adapted depending on the current activity pattern. In other words, the parameter values of the current activity pattern may be used to update the parameter values of the previous and/or averaged activity values. For example, new average values taking into account the parameter values of the current activity pattern may be determined.

According to an embodiment of the invention, the step of determining, whether there is an upcoming health issue of the user, may be carried out depending on the evaluation of the current activity pattern and additionally depending on at least one audio parameter. So, the decision, whether there might be the upcoming health issue or not, may be made not only depending on the current activity pattern but also depending on one or more audio parameters, in particular depending on audio parameter values of the corresponding audio parameters.

According to an embodiment of the invention, the audio parameter may represent at least in part a current acoustic environment of the user and/or information regarding the modified audio signal. For example, the audio parameter values of one of the audio parameters representing the current acoustic environment may be derived directly from the received audio signal, e.g. an acoustic level and/or frequency spectrum of the audio signal, or indirectly from the received audio signal, e.g. a classification value generated by a classifier of the hearing system depending on the received audio signal. The classifier may be arranged for classifying the acoustic environment of the user depending on the received audio signal, wherein the classification value may also be used for determining a sound program, which may be carried out by the sound processor for modifying the received audio signal. In contrast, the audio parameters representing the information regarding the modified audio signal may be derived from the modified audio signal itself, e.g. an acoustic level, content, and/or frequency spectrum of the modified audio signal.

Considering the audio parameter for the decision, whether there is the upcoming health issue or not, may take into account that activities can be more difficult for a cognitively ill user to perform the more distracted he or she is, e.g. by loud ambient noise, or the more things his or her brain has to cope with at the same time. For example, manual operation of the hearing device or any other everyday movement or speech behaviour may be even slower in a noisy environment than in a quiet environment. With the hearing device it is possible to detect the characteristics of the acoustic surroundings, e.g. the loudness, noise, etc. This may be done by the conventional classifiers or more recently by deep neuronal networks (DNNs) already known to the person skilled in the art. These characteristics may be correlated with the parameter values representing the current activity pattern to make the health issue prediction more reliable. So, in addition to the current activity pattern, which may represent a time-dependent diary of properties and/or activities of the user, an environmental noise-dependent diary may be generated by storing the parameter values of the audio parameters, wherein this diary may be considered for determining whether there is the upcoming health issue or not. For example, data that are informative for the environmental sounds could be fed as further input to the DNN.

Similarly, characteristics of the modified audio signal played back to the user, e.g. when playing back an ambient sound detected by the sound input component and/or when playing back audio signals received electronically, such as streamed audio data or a phone call, could be handled. However, the hearing device may compensate for certain environmental effects by running the different sound programs designed to make it easier for the user to hear in different environmental situations, e.g. in a "Speech in Noise" acoustic scene identified by the classifier, the ambient noise level may be reduced to make it easier for the user to carry on a conversation. This makes it more difficult to assess an effect of ambient noise on the cognitive performance of the user. For example, if the ambient noise is masked out, the cognitive performance may remain the same or even improve. Therefore, the activity data for generating the activity pattern may be collected separately for the sound programs that are running in each case, and/or the currently running sound program may be fed as another input to the DNN. Furthermore, it may be possible to record different characteristics of the environmental sounds within one sound program without switching to another sound program, but only as input for the determination of the cognitive characteristics of the user.

According to an embodiment of the invention, evaluating the current activity pattern may comprise: determining which health issue comes up based on previous activity patterns of unhealthy people having the corresponding health issue, and providing the predetermined early detection signal indicating the upcoming health issue, if it is determined that there is an upcoming health issue of the user. The corresponding health issue may be identified by the neuronal network. In this case, the neuronal network may have been trained with the activity patterns of persons having the corresponding health issue, or the current activity pattern may be compared with the activity patterns of persons having the corresponding health issue.

According to an embodiment of the invention, the health issue may refer to the health of the brain of the user. In particular, the health issue may be at least one of the group of dementia, Alzheimer's disease, and cognitive decline.

According to an embodiment of the invention, the activity patterns of the user, e.g. the current and/or previous activity pattern, each comprise parameter values of a set of parameters, wherein the parameters comprise personal parameters and/or social parameters.

According to an embodiment of the invention, the activity pattern of the user comprises parameter values of a set of parameters, wherein the parameters comprise at least one of the group of personal parameters and social parameters. The personal parameters may comprise at least one of the group of activity parameters indicative of a physical and/or mental activity performed by the user; physiological parameters indicative of a physiological property of the user; dexterity parameters indicative of a manual dexterity of the user; and speech parameters indicative of a speaking capability of the user. The social parameters may be indicative of a contact of the user to other people, whether, how often, and/or how long the user listens and/or talks to other people, and/or a number of people the user interacts with, and/or a frequency in which the user interacts with different people, and/or a time the user spent in different social situations, like quiet situations and/or situations involving other people speaking.

According to an embodiment of the invention, the method further comprises determining, based on the received audio signal generated by the sound detector (20), a sound environment pattern of the user, wherein said evaluating the current activity pattern comprises correlating the current activity pattern of the user with the sound environment pattern; determining, based on the received audio signal generated by the sound detector, a sound environment pattern of the user, wherein said evaluating the current activity pattern comprises correlating the current activity pattern of the user with the sound environment pattern. The method may further comprise receiving the audio signal generated by the sound detector within the predetermined time interval. The method may further comprise classifying the received audio signal generated by the sound detector by assigning the audio signal to a class from a plurality of predetermined classes, wherein the sound environment pattern of the user is determined based on the class assigned to the audio signal; and/or determining a volume level of the received audio signal generated by the sound detector, wherein the sound environment pattern of the user is determined based on the determined volume level. The method may further comprise determining a characteristic of the received audio signal generated by the sound detector, wherein the audio signal is assigned to the class depending on the determined characteristic. At least two of said classes may be associated with different audio processing parameters applied by the processing unit for modifying the audio signal.

According to an embodiment of the invention, the method further comprises monitoring the modifying of the audio signal within the predetermined time interval; determining, based on the monitored modifying of the audio signal, an audio output pattern of the hearing device, wherein said evaluating the current activity pattern comprises correlating the current activity pattern of the user with the audio output pattern.

According to an embodiment of the invention, the sound environment pattern of the user and/or the audio output pattern of the hearing device comprises parameter values of at least one parameter, wherein the parameter is indicative of a distraction level of the user. The distraction level may relate to the sound in the environment and/or the audio outputted by the hearing device.

According to an embodiment of the invention, the method further comprises determining a charging status of a battery included in the hearing device; determining, based on the charging status, a charging status pattern of the hearing device, wherein said evaluating the current activity pattern comprises correlating the current activity pattern of the user with the charging pattern.

According to an embodiment of the invention, the user detector comprises an own voice detector configured to detect an own voice activity of the user wherein the user detection signal may be indicative of the own voice activity; and/or a sound classifier configured to identify a sound class in the audio signal generated by the sound detector, wherein the sound class may be representative of an occurrence of a social activity of the user, wherein the user detection signal may be indicative of the sound class; and/or a movement sensor configured to detect a movement of the hearing device, wherein the user detection signal may be indicative of the movement; and/or a biometric sensor configured to detect a biometric property of the user, wherein the second detection signal may be indicative of the biometric property; and/or a user-interface configured to detect a user interaction, in particular a manual and/or vocal user interaction, wherein the user detection signal may be indicative of the user interaction; and/or a location detector configured to detect a location of the user and/or a change of location of the user, wherein the user detection signal may be indicative of the location; and/or a power manager configured to detect a charging status of a battery associated with the hearing device, wherein the user detection signal may be indicative of the charging status.

According to an embodiment of the invention, the personal parameters may refer to the user wearing the hearing device personally, and/or to any action carried out by the user. For example, the personal parameters may comprise at least one of the group of physical parameters, e.g. pulse, blood pressure, blood oxygen saturation, and/or body temperature; locomotory parameters, e.g. patterns and/or durations of, speeds of, and/or pauses between single or double taps, taps and hold, and/or swipes up/down of an input mean of the hearing system; and/or activity parameters, e.g. steps count, time spent in different activity levels, level of body movement and orientation; occurrence of head gestures like nodding or shaking; frequency and duration of listening to music, music styles, usage of the hearing device and/or a connected user device of the user, need for help menu in an App, and/or adjustments in the field.

According to an embodiment of the invention, the social parameters may refer to people except for the user, people interacting with the user, and/or people around the user. For example, the social parameters may refer to at least one of the group of a contact of the user to other people, whether, how often, and/or how long the user listens and/or talks to other people, and the time the user spent in different social situations, like quiet situations, noisy situations, and/or telephone calls.

An aspect of the invention relates to the hearing device, comprising the at least one sound detector for generating the audio signal, the at least one user detector for generating the detection signal, processing unit for modifying the audio signal, and the at least one sound output component for outputting the modified audio signal.

An aspect of the invention relates to a hearing system, comprising a hearing device, e.g. the above hearing device, and the user device connected to the hearing device for data exchange with the hearing device, wherein the hearing system is configured to carry out the above method. The method may be carried out within the hearing device, the connected user device, or in cooperation of both. The hearing system may further include, by way of example, a second hearing device worn by the same user.

An aspect of the invention relates to a computer program for early detection of a health issue of a user wearing the hearing device, the computer program, when being executed by a processor being adapted to carry out the steps of the above method. For example, the computer program may be executed in a processor of the hearing device. The computer-readable medium may be a memory of this hearing device. The computer program also may be executed by a processor of the connected user device, such as a smartphone or any other type of mobile device, which may be a part of the hearing system, and the computer-readable medium may be a memory of the connected user device. It also may be that some steps of the method are performed by the hearing device and other steps of the method are performed by the connected user device.

In general, a computer-readable medium may be a floppy disk, a hard disk, an USB (Universal Serial Bus) storage device, a RAM (Random Access Memory), a ROM (Read Only Memory), an EPROM (Erasable Programmable Read Only Memory) or a FLASH memory. A computer-readable medium may also be a data communication network, e.g. the Internet, which allows downloading a program code. The computer-readable medium may be a non-transitory or transitory medium.

According to an embodiment of the invention, the hearing device comprises: a microphone; a processor for processing a signal from the microphone; a sound output device for outputting the processed signal to an ear of the hearing device user; a transceiver for exchanging data with the connected user device and/or with another hearing device worn by the same user; and at least one classifier configured to detect and classify specific states of the user or of the user's environment which are relevant for the user's mood, based on at least one of: an audio signal from the at least one microphone or a sensor signal from at least one further sensor.

It has to be understood that features of the method as described above and in the following may be features of the controller, the hearing device, the hearing system, the computer program, and/or the computer-readable medium as described above and in the following, and vice versa.

These and other aspects of the invention will be apparent from and elucidated with reference to the embodiments described hereinafter.

### BRIEF DESCRIPTION OF THE DRAWINGS

Below, embodiments of the present invention are described in more detail with reference to the attached drawings.
Fig. 1 schematically shows a hearing device according to an embodiment of the invention.
Fig. 2 schematically shows a hearing system according to an embodiment of the invention.
Figs. 3 - 6 show flow diagrams of a method for early detection of a health issue of a user wearing a hearing device, according to some embodiments of the invention.
Figs. 7 and 8 show examples of a daily activity pattern of a user wearing a hearing device.
Figs.9 and 10 show examples of weekly activity patterns of a user wearing a hearing device.
Fig. 11 shows a schematic block diagram of a method for early detection of a health issue of a user wearing a hearing device, according to an embodiment of the invention.
Fig. 12 shows a schematic block diagram visualising at least in part an exemplary embodiment of a method for early detection of a health issue of a user wearing a hearing device, according to an embodiment of the invention.
Fig. 13 shows a schematic block diagram visualising exemplary embodiments of sensors and parameters usable for carrying out a method for early detection of a health issue of a user wearing a hearing device, according to an embodiment of the invention.
Fig. 14 shows a schematic block diagram visualising at least in part an exemplary embodiment of a method for early detection of a health issue of a user wearing a hearing device, according to an embodiment of the invention.

The reference symbols used in the drawings, and their meanings, are listed in summary form in the list of reference symbols. In principle, identical parts are provided with the same reference symbols in the figures.

### DETAILED DESCRIPTION OF EXEMPLARY EMBODIMENTS

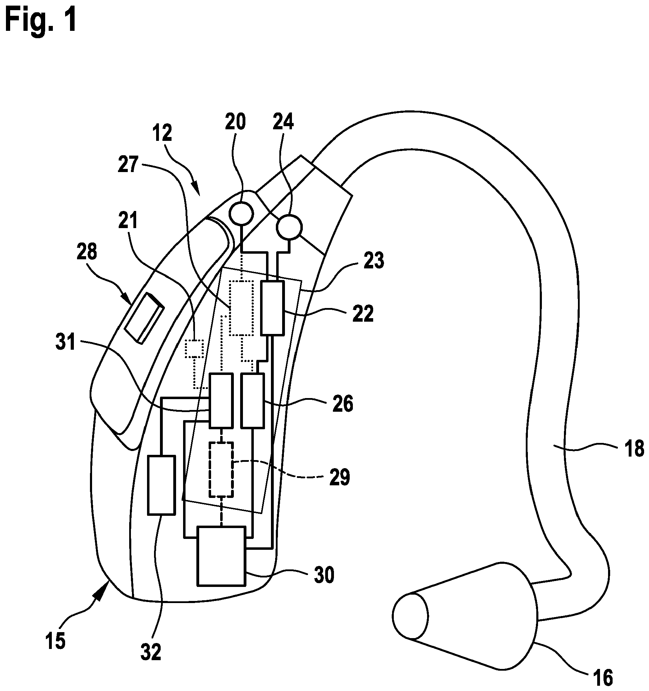

Fig. 1 schematically shows a hearing device 12 according to an embodiment of the invention. The hearing device 12 is formed as a behind-the-ear device carried by a hearing device user (not shown). It has to be noted that the hearing device 12 is a specific embodiment and that the method described herein also may be performed with other types of hearing devices, such as e.g. an in-the-ear device or one or two of the hearing devices mentioned above.

The hearing device 12 comprises a part 15 behind the ear and a part 16 to be put in the ear channel of the user. The part 15 and the part 16 are connected by a tube 18. In the part 15, at least one sound detector 20, e.g. a microphone or a microphone array, a processing unit 23 and a sound output component 24, such as a loudspeaker. The sound detector 20 can detect a sound in the environment of the user and generate an audio signal indicative of the detected sound. The processing unit 23 can receive the audio signal generated by the sound detector 20.

The hearing device 12 may include a sound processing module 22. For instance, the sound processing module 22 may be implemented in a computer program executed by the processing unit 21. The sound processing module 22 may be configured to modify, in particular amplify, dampen and/or delay, the audio signal generated by the sound detector 20, e.g. some frequencies or frequency ranges of the audio signal depending on parameter values of parameters, which influence the amplification, the damping and/or, respectively, the delay, e.g. in correspondence with a current sound program. The parameter may be one or more of the group of frequency dependent gain, time constant for attack and release times of compressive gain, time constant for noise canceller, time constant for dereverberation algorithms, reverberation compensation, frequency dependent reverberation compensation, mixing ratio of channels, gain compression, gain shape/amplification scheme. A set of one or more of these parameters and parameter values may correspond with a predetermined sound program, wherein different sound programs are characterized by correspondingly different parameters and parameter values. The sound program may comprise a list of sound processing features. The sound processing features may for example be a noise cancelling algorithm or a beamformer, which strengths can be increased to increase speech intelligibility but with the cost of more and stronger processing artifacts. The sound output component 24 can generate sound from the modified audio signal and the sound can be guided through the tube 18 and the in-the-ear part 16 into the ear channel of the user. at least one sensor 21, e.g. an accelerometer, an optical, or a temperature sensor, are provided

The hearing device 12 may include a sound processing control module 26. For instance, the sound processing control module 26 may be implemented in a computer program executed by the processing unit 21. The sound processing control module 26 may be configured for adjusting parameters of the sound processing module 22, e.g. such that an output volume of the sound signal is adjusted based on an input volume. These parameters may be determined by a computer program which is referred to as a sound program. For example, with an input mean 28, e.g. a knob, a button, or a touch-sensitive sensor, e.g. capacitive sensor, of the hearing device 12, a user may select a modifier (such as bass, treble, noise suppression, dynamic volume, etc.) and levels and/or values of these modifiers may be selected, from this modifier, an adjustment command may be created and processed as described above and below. In particular, processing parameters may be determined based on the adjustment command and based on this, for example, the frequency dependent gain and the dynamic volume of the sound processing module 22 may be changed. All these functions may be implemented as different sound programs stored in a memory 30 of the hearing device 12, which sound programs may be executed by the sound processing module 22. The memory 30 may be implemented by any suitable type of storage medium, in particular a non-transitory computer-readable medium, and can be configured to maintain, e.g. store, data controlled by the processing unit 23, in particular data generated, accessed, modified and/or otherwise used by the processing unit 23. The memory 30 may also be configured to store instructions for operating the hearing device 12 and/or a connected user device that can be executed by the processing unit 23, in particular an algorithm and/or a software that can be accessed and executed by the processing unit 23.

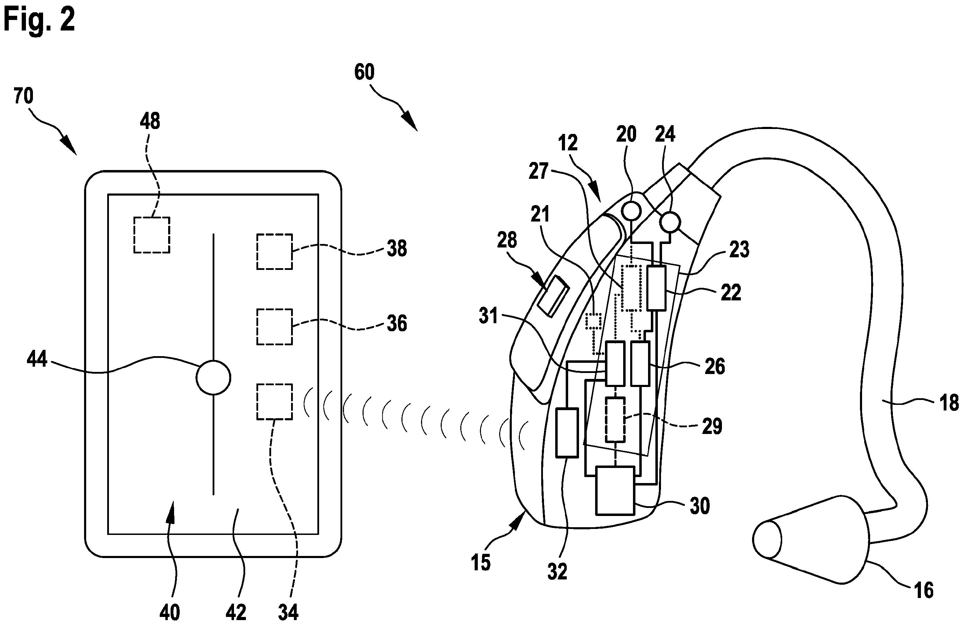

The hearing device 12 may further comprise a transceiver 32. The transceiver 32 may be configured for wireless data communicated with a remote server. Additionally or alternatively, the transceiver may be a first transceiver 32 which may be adapted for wireless data communication with a second transceiver 34 of a connected user device 70 (see figure 2). The first and/or the second transceiver 32, 34 each may be e.g. a Bluetooth or RFID radio chip.

The hearing device 12 further comprises a user detector for generating a user detection signal indicative of a property of the user and/or an activity performed by the user. The user detection signal can be representative of different sets of personal data, in particular user-related data. As illustrated, the user detector may comprise an audio signal evaluation module 27 and/or a sensor 21 and/or a user interface, for instance the input mean 28. The user detector may comprise other components, in particular modules and/or sensors, suitable for generating a user detection signal.

As illustrated, the audio signal evaluation module 27 may be implemented in a computer program executed by the processing unit 21. The audio signal evaluation module 27 can be configured to evaluate the audio signal generated by the sound detector 20. In some implementations, the audio signal evaluation module 27 comprises a sound classifier configured to classify the audio signal generated by the sound detector 20 by assigning the audio signal to a class from a plurality of predetermined classes. To this end, the sound classifier 27 may be configured to determine a characteristic of the audio signal generated by the sound detector 20, wherein the audio signal is assigned to the class depending on the determined characteristic. For instance, the sound classifier 27 can be configured to identify one or more predetermined classification values based on the audio signal from the sound detector 20. The classification may be based on a statistical evaluation of the audio signal and/or a machine learning (ML) algorithm that has been trained to classify the ambient sound.

To illustrate, the sound classifier 27 may be configured to identify at least one signal feature in the audio signal generated by the sound detector 20, wherein the characteristic determined from the audio signal corresponds to a presence and/or absence of the signal feature. Exemplary characteristics include, but are not limited to, a mean-squared signal power, a standard deviation of a signal envelope, a mel-frequency cepstrum (MFC), a mel-frequency cepstrum coefficient (MFCC), a delta mel-frequency cepstrum coefficient (delta MFCC), a spectral centroid such as a power spectrum centroid, a standard deviation of the centroid, a spectral entropy such as a power spectrum entropy, a zero crossing rate (ZCR), a standard deviation of the ZCR, a broadband envelope correlation lag and/or peak, and a four-band envelope correlation lag and/or peak. For example, the sound classifier 27 may determine the characteristic from the audio signal using one or more algorithms that identify and/or use zero crossing rates, amplitude histograms, auto correlation functions, spectral analysis, amplitude modulation spectrums, spectral centroids, slopes, roll-offs, auto correlation functions, and/or the like. In some instances, the characteristic determined from the audio signal is characteristic of an ambient noise in an environment of the user, for instance a noise level, and/or a speech, for instance a speech level. The audio signal analyzer may be configured to divide the audio signal into a number of segments and to determine the characteristic from a particular segment, for instance by extracting at least one signal feature from the segment. The extracted feature may be processed to assign the audio signal to the corresponding class.

The sound classifier 27 may be further configured to assign, depending on the determined characteristic, the audio signal generated by the sound detector 20 to a class of at least two predetermined classes. The characteristic may be processed to assign the audio signal to the corresponding class. The classes may represent a specific content in the audio signal. For instance, the classes may relate to a speaking activity of the user and/or another person and/or an acoustic environment of the user. Exemplary classes include, but are not limited to, low ambient noise, high ambient noise, traffic noise, music, machine noise, babble noise, public area noise, background noise, speech, nonspeech, speech in quiet, speech in babble, speech in noise, speech in loud noise, speech from the user, speech from a significant other, background speech, speech from multiple sources, calm situation and/or the like. In some instances, the classifier is configured to evaluate the characteristic relative to a threshold. The classes may comprise a first class assigned to the audio signal when the characteristic is determined to be above the threshold, and a second class assigned to the audio signal when the characteristic is determined to be below the threshold. For instance, when the characteristic determined from audio signal 401 is characteristic of an ambient noise, a first class representative of a high ambient noise may be assigned to the audio signal when the characteristic is above the threshold, and a second class representative of a low ambient noise may be assigned to the audio signal when the characteristic is below the threshold. As another example, when the characteristic determined from the audio signal is characteristic of a speech, a first class representative of a larger speech content may be assigned to the audio signal when the characteristic is above the threshold, and a second class representative of a smaller speech content may be assigned to the audio signal when the characteristic is below the threshold. Thus, from the classes assigned to the audio signal, activity parameters can be derived which can be indicative of a physical and/or mental activity performed by the user and/or social parameters can be derived which can be indicative of a contact of the user to other people, whether, how often, and/or how long the user listens and/or talks to other people, and/or a number of people the user interacts with, and/or a frequency in which the user interacts with different people. To illustrate, a class related to traffic noise may indicate a traveling activity of the user, a class related to the user's speech may indicate a speaking activity of the user, and a class related to speech from a significant other and/or speech from multiple sources may indicate a contact of the user to other people.

The sound classifier 27 may be further configured to classify an audio signal different from the audio signal generated by the sound detector 20 by assigning the different audio signal to a class from a plurality of predetermined classes. For instance, the different audio signal may be a streamed audio signal which may be received via transceiver 32. The classes may then represent a specific content in the streamed audio signal. Exemplary classes include, but are not limited to, streamed music, streamed speech, phone calls, speech received from a remote microphone, e.g. a remote microphone worn by a significant other and/or the like.

At least two of the classes can be associated with different audio processing parameters, in particular different sound programs, which can be applied by the sound processor 22 for modifying the audio signal. To this end, the class assigned to the audio signal, which may correspond to a classification value, may be provided to the sound processing control module 26 in order to select the associated audio processing parameters, in particular the associated sound program, which may be stored in the memory 30. The class assigned to the audio signal may thus be used to determine the sound program, which may be automatically used by the hearing device 12, in particular depending on the audio signal received from the sound detector 20 and/or an audio signal received from the transceiver 32.

The sensor 21 may comprise at least one of the group of a movement sensor, a biometric sensor, a location detector, and an own voice detector. The movement sensor be implemented by any suitable device configured to provide movement data indicative of a movement of the hearing device 12, in particular of the user wearing the hearing device. The movement sensor can include an inertial sensor, for instance an accelerometer configured to provide the movement data representative of an acceleration and/or displacement and/or rotation of the hearing device, and/or a gyroscope configured to provide the movement data representative of a rotation of the hearing device. The movement sensor may also include a magnetometer, in particular an electronic compass, configured to measure the direction of an ambient magnetic field. A movement property of the user may be determined based on the movement data, e.g. by the sensor 21 itself and/or by processing unit 23 receiving the movement data. A physical activity performed by the user can correspond to any movement property of the user, which may be determined based on the movement data. In particular, the movement property may be determined based on a predetermined pattern in the movement data indicative of the movement property. To illustrate, the movement property can include rather simple and/or short term movements of the user, such as standing up, sitting down, lying down, walking, running, and/or head turns and/or rather complex and/or long term movements of the user, such as dancing, bicycle riding, brushing teeth, cooking, eating, vacuum cleaning, and/or the like. To illustrate, at least in the case of the rather simple and/or short term movements of the user, the predetermined pattern may be identified in the movement data based on predefined signal features and/or thresholds, such as an amplitude, slope, and/or a frequency of zero-crossings and/or inflection points over time in the movement data. For instance, the user standing still may be distinguished from the user walking and/or the user walking may be distinguished from the user running by a lower frequency of zero-crossings over time and/or a lower frequency of inflection points over time in the movement data with respect to a movement direction of the hearing device perpendicular to the surface of the earth. The predetermined pattern of those movements and also a predetermined pattern of more complex and/or long term movements can also be identified based on a trained machine learning algorithm which may be configured, for instance, to classify the movement data with respect to the different movements of the user. Thus, from the movement data provided by the movement sensor, activity parameters indicative of a physical activity performed by the user and/or social parameters can be derived. To illustrate, movement data indicating that the user is engaged in a team sport, such as playing volleyball, can be attributed to a social activity of the user.

The biometric sensor may be implemented by any suitable device configured to provide biometric data indicative of a biometric property of the user. The biometric property can include, for instance, a heart rate, resting heart rate (RHR), heart rate recovery time, heart rate variation and/or arrhythmia, blood pressure, elevated blood pressure (hypertension), saturation pressure of blood oxygen (SpO2), maximum oxygen consumption (VO₂ max), blood glucose level, blood alcohol level, blood composition, cardiovascular health, endurance level, aerobic fitness level, biorhythm, body temperature, sweat rate, respiratory rate, cognitive load, listening intention, listening effort, breakdown of neural activity over time, sleeping or waking state, distraction level, concentration level, relaxation level, physical exhaustion level, physiological stress, and/or the like. In some examples, the biometric sensor comprises a photoplethysmography (PPG) sensor and/or an electrocardiography (ECG) sensor and/or an electroencephalography (EEG) sensor and/or an electrooculography (EOG) sensor and/or a temperature sensor and/or a skin conductance sensor and/or a radio frequency (RF) sensor and/or a pupillometry sensor. The biometric sensor may be configured to provide an acute measurement of a biological characteristic, for instance by directly detecting energy and/or matter from a living organism, and/or a processed collection of acute measurements of the biological characteristic, for example by recording the biological characteristic over time such as in a PPG waveform and/or a recorded EEG signal. Biometric data may be any data provided by the biometric sensor representative for the biological characteristic. A physiological property of the user may be determined based on the biometric data, e.g. by the sensor 21 itself and/or by processing unit 23 receiving the biometric data. For instance, a heart rate and/or blood pressure and/or heart rate variability (HRV) and/or VO₂ max and/or aerobic fitness level and/or blood content and/or level of fatigue may be determined based on biometric data provided by a PPG sensor and/or an ECG sensor. A certain value range of at least one of those parameters, for instance the heart rate, may also indicated a physical activity of the user. A mental activity performed by the user, for instance a cognitive load and/or listening effort and/or concentration level and/or biorhythm and/or physiological stress and/or distraction level and/or a level of fatigue may be determined based on biometric data provided by an EEG sensor and/or a pupillometry sensor. An eye gaze movement may be determined based on biometric data provided by an EOG sensor. A body temperature may be determined based on the biometric data provided by a temperature sensor.

The location detector may be implemented by any suitable device configured to provide location data indicative of a change of the location of the user. For instance, the location sensor may include a navigation sensor such as a GPS sensor. Changing a location by the user can indicate a physical activity of the user.

The own voice detector may be implemented by any suitable device configured to detect an own voice activity of the user and to provide an own voice detection signal indicative of the own voice activity. In particular, the own voice detector may comprise a voice activity detector (VAD) configured to determine whether an own voice activity of the user is present or absent. For instance, the own voice detector may be based on picking up the user's voice transmitted from the user's vocal chords to the ear canal wall via bone conduction through the user's head. For this purpose, a bone conductive microphone and/or a pressure sensor may be employed inside the ear canal to probe the bone conducted signal. The own voice detector may also be based on a signal analysis of the audio signal generated by the sound detector 20 based on sound detected outside the ear canal in the ambient environment of the user. In this case, in addition to or in place of implementing the own voice detector in sensor 21, the own voice detector may be implemented in audio signal evaluation module 27, for instance as an own voice detection module configured to determine an own voice activity of the user in the audio signal generated by the sound detector 20. In some implementations, the own voice detector can be configured for speech recognition of the user's speech, for instance for a keyword recognition and/or a recognition of full sentences spoken by the user. The own voice detection signal, which may be based on VAD and/or speech recognition, may also be employed to determine a variety of own voice characteristics of the user, which may be related to any of the group of linguistic features, an occurrence of speech and pauses in speech, a signal level, a frequency range, and a temporal variation of the own voice activity, in a distinguishable manner. The linguistic features can comprise an occurrence of at least one of vowels, consonants, voiced phonemes, and unvoiced phonemes in the own voice activity and/or an occurrence of at least one of spoken words and phrases in the own voice activity. To illustrate, the occurrence of at least one linguistic feature can comprise a number and/or frequency of at least one of vowels, consonants, voiced phonemes, and unvoiced phonemes occurring in the own voice activity and/or a number and/or frequency of at least one of spoken words and phrases occurring in the own voice activity. The occurrence of speech and pauses in speech can include a duration of the speech and the speech pauses. Thus, from the own voice detection signal, speech parameters indicative of a speaking capability of the user may be derived.

The user interface is configured to detect a user interaction with the hearing device. For instance, the user interface can comprise input mean 28 by which the user can manually interact with the hearing device 12. The user interface can also comprise speech recognition, in particular by an own voice detector, by which the user can vocally interact with the hearing device 12, for instance by means of a keyword which can be recognized as a command to be executed by the hearing device 12. The user interface can thus be configured to provide user interaction data which may be indicative, for instance, of a frequency at which the user interacts with the hearing device 12, a number of times in which the user performs specific user interactions, which may include redundant and/or nonsensical user interactions, a performance of the user when interacting with the hearing device, for instance a duration required by the user for the user interaction and/or a delay of the user to perform a user interaction after a specific event requiring the user interaction and/or a failure of the user when trying to perform a specific user interaction, and/or the like. A manual interaction of the user with the user interface 28, such as tapping, swiping or typing, can indicate a dexterity of the user, for instance by determining a duration of a pause between two subsequent manual interactions. Thus, from the user interaction data provided by the user interface, dexterity parameters indicative of a manual dexterity of the user, in particular a difficulty of performing certain manual operations, may be derived.

The hearing device may further comprise a power manager 29. The power manager 29 may comprise a power management module which may be implemented in a computer program executed by the processing unit 21. The power manager 29 can be configured to detect a charging status of a battery, in particular a rechargeable battery, included in the hearing device 12. For instance, the charging status can be indicative of whether the battery is currently discharging or recharging and/or whether the hearing device 12 is placed outside of a battery charger or inside a battery charger, how often and/or how much the battery is recharged and/or discharged, how intensively the hearing device is used as indicated by an amount of the power consumption, how long the hearing device 12 is not used resulting in a rather low power consumption of the battery, how long the hearing device 12 is left in the battery charger after the battery is fully charged, a time and/or frequency of the recharging, and/or the like. Thus, from the charging status detected by the power manager 29, a charging pattern of the hearing device 12 can be derived. For instance, the charging pattern can include parameters related to the hearing device 12 being placed in a battery charger and/or the hearing device 12 being worn and/or operated by the user, which may be indicated by a larger amount of discharging, and/or not used, which may be indicated by a smaller amount of discharging.

The hearing device 12 may further comprise an environmental detector configured to detect a property of the ambient environment of the user. For instance, the property detected in the ambient environment can include a sound in the environment, such as a sound level and/or voices of people surrounding the user and/or traffic noise, ambient temperature, humidity, barometric pressure, altitude, weather, climate, solar energy, wind, smog, rain, hail, snow, ice, daylight, darkness, visibility conditions, airborne particle density, pollution, traffic, exhaust from vehicle engines, and/or the like. The environmental detector may comprise the audio signal evaluation module 27, in particular the sound classifier. In particular, as described above, the sound classifier 27 can be configured to classify the audio signal generated by the sound detector 20 in classes related to an activity performed by the user and/or in classes related to a property of the ambient environment. For example, classes indicative of a property of the environment can be representative of an amount of environmental noise, such as low ambient noise and high ambient noise, and/or a type of the environmental noise, such as machine noise, babble noise, and public area noise. The environmental detector may also comprise any other sensor suitable to detect a property of the ambient environment, for instance a location sensor indicating a momentary location of the user. Further, in some implementations, the audio signal modified by the sound processor 22 before the audio signal is outputted to the user by the sound output component 24 can be employed as environmental data indicative of the ambient environment of the user. To illustrate, the outputted sound waves can be perceived by the user as a constituent part of the ambient environment and/or influence the perception of the user of the ambient environment. For instance, the sound processing control module 26 and/or the sound processor 22 may be employed to provide information about the audio signal modified by the sound processor 22. This information may be monitored for a predetermined time interval and an audio output pattern of the hearing device 12 can then be provided based on the monitored information.

The hearing device 12 may include a health issue determining control module 31. For instance, the health issue determining control module 31 may be implemented in a computer program executed by the processing unit 21. The health issue determining control module 31 can be configured to determine a current activity pattern of the user by evaluating the audio signal received by the processing unit 23 over a predetermined time interval. The current activity pattern of the user may then be evaluated by the health issue determining control module 31 and/or a remote device. For instance, the remote device may be a user device connectable to the hearing device and/or a remote server connectable to the hearing device and/or the remote device. For instance, the health issue determining control module 31 may be configured to transmit the current activity pattern of the user to the remote device, e.g. by means of the transceiver 32. Depending on the evaluation, it may be determined, by the health issue determining control module 31 and/or the remote device, whether there is an upcoming health issue of the user. Then, if it is determined that there is an upcoming health issue of the user, an output signal indicative of the health issue may be provided. In some instances, the output signal may be predetermined, in particular before the current activity pattern of the user is determined. For instance, the health issue determining control module 31 and/or the remote device can be configured to initiate the providing of the output signal. For instance, the output signal may be provided to the user, in particular by the hearing device and/or a user device, and/or a device of another person, for instance a significant other of the user and/or a health professional. Providing the output signal may comprise outputting an audio message and/or providing an electronic message which can be displayed on a display. Those and other implementations are further described in the description that follows.

Each of the sound processing module 22, the sound processing control module 26, the audio signal evaluation module 27, the power manager 29, and the health issue determining control module 31 may be embodied in hardware or software, or in a combination of hardware and software. Further, at least two of the modules 22, 26, 27, 29, 31 may be consolidated in one single module or may be provided as separate modules. The processing unit 23 may be implemented as a single processor or as a plurality of processors. For instance, the processing unit 21 may comprise a first processor in which the sound processing module 22 is implemented, and a second processor in which the sound processing control module 26 and/or the audio signal evaluation module 27 and/or the power manager 29 and/or the health issue determining control module 31 are implemented.

The hearing device 12 is thus configured for early detection of a health issue of the user wearing the hearing device 12, according to the present invention.

Fig. 2 schematically shows a hearing system 60 according to an embodiment of the invention. The hearing system 60 includes a hearing device, e.g. the above hearing device 12, and a connected user device 70, such as a smartphone or a tablet computer. The connected user device 70 may comprise the second transceiver 34, a processing unit 36, a memory 38, a graphical user interface 40 and a display 42. Alternatively or additionally to the sound classifier 27 of the hearing device 12, the connected user device 70 may comprise a sound classifier 48, which may be implemented in a computer program executed by the processing unit 36. If the hearing device 12 is controlled via the connected user device 70, the processing unit 36 of the user device 70 may be seen at least in part as a controller of the hearing device 12. In other words, according to some embodiments, the processing unit 23 of the hearing device 12 and the processing unit 36 of the user device may form the controller of the hearing device 12. A processing unit of the hearing system 60 may comprise the processing unit 23 of the hearing device 12 and the processing unit 36 of the user device 70. Processing units 23, 36 may communicate data via transceivers 32, 34.

With the hearing system 60 it is possible that the above-mentioned modifiers and their levels and/or values are adjusted with the connected user device 70 and/or that the adjustment command is generated with the connected user device 70. This may be performed with a computer program run in the processing unit 36 and stored in the memory 38 of the user device 70. The computer program may provide the graphical user interface 40 on the display 42 of the connected user device 70. For example, for adjusting the modifier, such as volume, the graphical user interface 40 may comprise a control element 44, such as a slider. When the user adjusts the slider, an adjustment command may be generated, which will change the sound processing of the hearing device 12 as described above and below. Alternatively or additionally, the user may adjust the modifier with the hearing device 12 itself, for example via the input means 28. The user interface 40 of the user device 70 may be employed to provide user interaction data to the health issue determining control module 31 as described above with regard to the user interface 28 of the hearing device 12.

The user device 70 may also comprise a sensor to provide other user detection data, which may comprise at least one of the group of a movement sensor, a biometric sensor, a location detector, and an own voice detector, as described above in conjunction with the sensor 21 of the hearing device 12. At least part of the user detection data is preferably provided by the hearing device 12, wherein additional user detection data may be provided by the user device 70. In particular, the user detection data provided by the hearing device 12 can be employed to provide the activity pattern of the user in a more reliable and/or conclusive way as compared to the user detection data provided by the user device 70, since the hearing device 12 may typically be used by the user for a longer uninterrupted time interval at a more steady body position as compared to the user device 70. Moreover, the wearing position of the hearing device 12 at the user's ear can be more favorable for collecting user detection data that would be significant for the determining of the activity pattern based on which a health issue could be determined. In particular, the position of the hearing device 12 rather close to the user's brain, mouth, and sensory organs can allow to collect a plethora of user detection data representative of the user's activities, physiological properties, dexterity, speech, and perception of social activities, allowing to draw more reliable conclusions with regard to the user's health.

In some implementations, the health issue determining control module 31 may be implemented in the user device 70, in particular as a computer program executed by the processing unit 36 of the user device 70. A user detection signal indicative of a property of the user and/or an activity performed by the user may then be received by the processing unit 36 of the user device 70 from the hearing device 12 via transceivers 32, 34. In some implementations, the health issue determining control module 31 may be implemented in both the hearing device 12 and the user device 70, in particular as a computer program executed by the processing unit of the hearing system 60, which may comprise processing units 23, 36.

On the hearing device 12 and/or user device 70, a user detection signal representative of different sets of personal data may thus be produced, as described above. Some further examples may include: with the sensor 21, in case it is an accelerometer or gyroscope, physical activity data, such as steps count of the user and/or time the user spent in different activity levels, may be generated; with the input mean 28 a user interaction behavior and/or locomotory capabilities may be monitored and a corresponding interaction or activity pattern may be generated, e.g. by monitoring and/or evaluating single or double taps, tap and hold, and/or swipe up/down of the input mean 28; with the classifier 27, 48 a social activity of the user may be monitored, e.g. time the user spent in different social situations; with the transceiver 32, 34 additional configuration interactions may be monitored, such as need for help menu in an App and/or adjustments in the field, phoning, e.g. need for making calls and/or patterns of callers, and/or audio streaming, e.g. a frequency and/or duration of listening to music and/or music styles; with a power management of the hearing system 60, e.g. the hearing device 12, a battery monitoring and/or on/off-times of the hearing device 12 may be monitored. All this information and the corresponding data may be stored in the hearing system 60, e.g. the hearing device 12. Through a mobile connection the data may be transferred completely or in part from the hearing device 12 to the hearing system 60 for evaluating the data. In addition, further data may be obtained for data fusion and analysis from the connected user device 70.

From this data a daily activity pattern may be constructed individually for the user and may be stored over days, weeks, months, or years. Thus a daily, weekly, 20-day-, monthly, and/or yearly activity pattern may be generated. These individual activity patterns may be evaluated and/or may be compared with previous and/or older activity patterns of the user and/or with a reference dataset for comparison and/or benchmarking. The reference dataset may be the dataset of any healthy person, of the user in a healthy state, or the dataset of an unhealthy person, e.g. of a person having a given health issue. Possible embodiments may apply machine learning (e.g. a neural network, e.g. a deep neural network) for these tasks. For example, activity patterns from healthy, respectively, unhealthy datasets (showing a specific disease pattern) may be learnt through machine learning techniques and individual activity patterns of users can be classified as healthy, respectively, unhealthy using the trained models. Optionally, the classes may be defined more specific, in particular the class of "unhealthy" activity patterns. In particular, there may be two or more classes of "unhealthy" activity patterns, wherein the different classes may refer to different health issues. In this case, these further classes may be learned from training with corresponding datasets of "unhealthy" activity patterns of people having the corresponding health issue. The data itself may be (pre-)processed and analyzed by using unsupervised learning, such as clustering methods (e.g. k-means clustering).

If a current activity pattern of the user matches the "healthy dataset", i.e. the dataset of a healthy person, it may be determined that there is no health issue. If the current activity pattern matches an "unhealthy dataset", i.e. the dataset of an unhealthy person, it may be determined that there is a health issue. In particular, the current activity pattern, i.e. the corresponding dataset, may be compared to a typical dataset of a person having a given health issue, e.g. Alzheimer's disease, general cognitive decline, or other mental health issues. For example, the sundown syndrome may be easily recognized from monitoring the activity and from the corresponding activity patterns, wherein the sundown syndrome may be a main indicator for the above diseases and may be detected straight forwardly at a very early stage of the corresponding disease.

So, the social behavior and/or physical behavior and/or physiological properties and/or mental activities and/or dexterity properties and/or speech capabilities of the user may be monitored, and the corresponding data may be collected at the ear of the user to reconstruct the current, e.g. daily, activity pattern. This current activity pattern may be evaluated in order to recognize decreasing brain health, e.g. dementia, Alzheimer's disease, or cognitive decline, in an early stage.

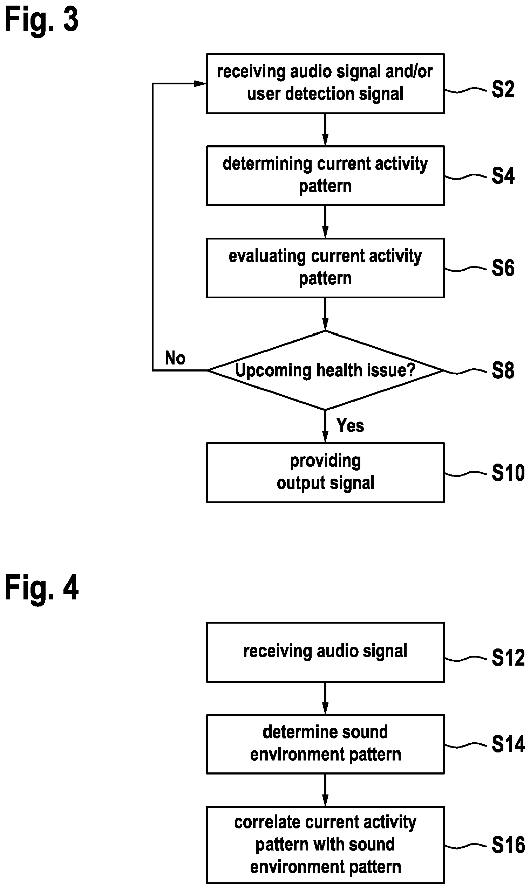

Fig. 3 shows a flow diagram of a method for early detection of a health issue of a user wearing a hearing device, e.g. the above hearing device 12, according to an embodiment of the invention. The method may be a computer-implemented method performed automatically in the hearing device 12 and/or the hearing system 60.

In step S2, an audio signal of the sound detector 20 and/or a user detection signal of the user detector may be received as an input signal for a predetermined time interval. The predetermined time interval may be e.g. 12 or 24 hours, several days, e.g. one week or one month. The corresponding data may be stored in the memory 30 of the hearing device 12 and/or the memory 38 of the user device 70. In some instances, in step S2, all input data, e.g. personal data, which may be included in the audio signal and/or the user detection signal, and environmental data, may be received, which may include audio data in the audio signal, such as data related to the acoustic environment for further processing by the hearing system 60, in particular information about a class associated with the audio signal generated by the sound detector 20 which may be provided by one of the sound classifiers 27, 48.

In step S4, a current activity pattern of the user is determined by evaluating the input signal received over the predetermined time interval, in particular by evaluating the corresponding data. In other words, the current activity pattern is generated based on the personal data of the user collected over the predetermined time interval. In this context, the current activity pattern may be referred to as daily, weekly, 20-day, or, respectively, monthly activity pattern, wherein in principle any given time interval from 12 hour to 3 years may be meaningful. The activity patterns, e.g. current, previous, daily, weekly, 20-day, and/or monthly activity patterns, of the user each may comprise parameter values of a set of parameters, wherein the parameters may comprise personal parameters and/or social parameters and wherein any of the above mentioned personal data or sets of personal data produced in the hearing device 12 may be used. The personal parameters may comprise at least one of the group of activity parameters indicative of a physical and/or mental activity of the user and/or physiological parameters indicative of a physiological property of the user and/or dexterity parameters indicative of a manual dexterity of the user and/or speech parameters indicative of a speaking capability of the user.. The social parameters may refer to at least one of the group of a contact of the user to other people, whether, how often, and/or how long the user listens and/or talks to other people, a number of people the user interacts with, a frequency in which the user interacts with different people, and a time the user spent in different social situations, like quiet situations and/or situations involving a speech of other people. In general, in step S4, the current activity pattern, what may be referred to as activity diary, may be determined depending on the received data by processes for recognizing and reconstructing of activities and the corresponding patterns.

In step S6, the current activity pattern may be evaluated. For example, the current activity pattern may be evaluated by a neural network, which has been trained in advance to differentiate an activity pattern of a healthy person from an activity pattern of an unhealthy people. Alternatively, the current activity pattern may be evaluated by comparing the current activity pattern with at least one previous activity pattern of the user. This also may be carried out by a corresponding neural network or by a "classical" algorithm. The previous activity pattern may represent an average of previously determined and stored activity patterns of the user. Alternatively or additionally, the current activity pattern may be evaluated by comparing the current activity pattern with a predetermined amount of previous activity patterns of the user. In general, in step S6, the resulting current activity pattern may be analysed and evaluated, e.g. by comparison with a predetermined benchmark, e.g. an activity pattern of a healthy or unhealthy person, e.g. by using machine learning or deep learning, e.g. algorithm ML2.

In step S8, it may be determined whether there is an upcoming health issue of the user depending on the evaluation in step S6. This step may be carried out by the neural network, which has been trained in advance to differentiate an activity pattern of a healthy person from an activity pattern of an unhealthy people. Alternatively or additionally, if the current activity pattern is evaluated by comparing the current activity pattern with the previous activity pattern, it may be determined that there is an upcoming health issue of the user if the current activity pattern differs from the previous activity pattern in a predetermined way. For example, a difference between the current activity pattern and the previous activity pattern may be determined by determining differences between parameter values of parameters constituting the current activity pattern and parameter values of parameters constituting the previous activity pattern. If the difference between one or more parameter values or an average of one or more parameter values exceeds a corresponding threshold, it may be determined that there is an upcoming health issue. In general, in step S8, it is decided, e.g. by comparison against a predetermined threshold, whether there is sufficient evidence for a conclusion regarding the presence of a health issue of the user. Optionally, in step S8, it may be determined which health issue comes up based on previous activity patterns of unhealthy people having the corresponding health issue. The health issue may refer to the health of the brain of the user. For example, the health issue is at least one of the group of dementia, Alzheimer's disease, cognitive decline.

In step S10, an output signal, for instance a predetermined early detection signal, may be provided, if it is determined that there is an upcoming health issue of the user in step S8. If it is determined which health issue of the user may come up in step S8, the predetermined early detection signal may concretely indicate the upcoming health issue. In general, in step S10, the final result of the conclusion of step S8 is presented as an output signal. The recipient of the output signal may be the user and/or to another person. The output signal may be outputted as an audio message and/or an electronic message which may be reproducible on a display to be read by the recipient.

Optionally, after evaluating the current activity pattern, the previous activity pattern may be adapted depending on the current activity pattern. For example, after the evaluation, the current activity pattern may be handled as previous activity pattern. Alternatively, the current activity pattern may be used to update or recalculate the previous activity pattern. For example, if the previous activity pattern is based on averaged parameter values of former current activity patterns, the parameter values of the current activity pattern may be used to calculate new averaged parameter values and as such a new previous activity pattern.

So, with the hearing device 12, which may be included in the hearing system 60, a smooth data collection and evaluation can be realised, which may take advantage of rather constant and/or reproducible measurement conditions over a long period of time during which the hearing device 12 is typically worn by the user at a similar wearing position it his ear. In particular, the hearing device 12 may be used as a consistent monitoring setup and may generate a continuous stream of activity data over a long period of time, e.g. over days, weeks, months, and years.

Fig. 4 shows a flow diagram of a method for early detection of a health issue of a user wearing a hearing device, e.g. the above hearing device 12, according to an embodiment of the invention, which may be implemented in conjunction with the method illustrated in Fig. 3. The method may be a computer-implemented method performed automatically in the hearing device 12 and/or the hearing system 60. In step S12, the audio signal generated by the sound detector 20 is received within the predetermined time interval in which the input signal is received in step S2. In particular, the audio signal received at S12 may be part of the input signal received in step S2 or may be received separately from the input signal received in step S2.

In step S14, a sound environment pattern of the user is determined based on the audio signal received in step S12. The sound environment pattern can represent properties of the sound in the ambient environment of the user at different times. The different times can be associated with corresponding times of the personal parameters and/or social parameters in the current activity pattern of the user. For instance, the sound environment pattern may comprise parameter values indicative of a distraction level of the user by the sound in the environment. Step S14 may include classifying the audio signal received at step S12 by assigning the audio signal to a class from a plurality of predetermined classes and/or determining a volume level of the audio signal received at step S12, which may be performed by the processing unit 23, 36, in particular the sound classifier 27, 48. Classifying the audio signal may include determining a characteristic of the audio signal, wherein the audio signal is assigned to the class depending on the determined characteristic. At least two of the classes may be associated with different audio processing parameters which may be applied by the processing unit 23, in particular the sound processing module 22, for modifying the audio signal, which may be controlled by the sound processing control module 26.

In step S16, the current activity pattern determined in step S4 is correlated with the sound environment pattern. The evaluating the current activity pattern in step S6 can include step 16. For example, the current activity pattern may be correlated with the sound environment pattern by inputting the sound environment pattern in addition to the current activity pattern in a neural network, for instance by using machine learning or deep learning, e.g. algorithm ML2. The neural network may have been trained in advance to differentiate an activity pattern of a healthy person from an activity pattern of an unhealthy people by also taking into account the sound environment pattern associated with the respective activity pattern. In this way, the evaluating the current activity pattern in step S6 can be performed in a more reliable way, and the determining whether there is an upcoming health issue of the user in step S8 can be more accurate.

To illustrate, any activities, which may be associated with the personal parameters and/or social parameters of the activity pattern, can be more difficult performed by a person the more the person is distracted, e.g. by loud ambient noises or specific audio sources in the environment, or the more things the person's brain has to deal with at the same time. This effect can be strongly enhanced by a person suffering from cognitive decline. For example, a manual operation performed on a user interface and/or a sequence of movements and/or a speaking behavior could be more negatively affected in a noisy environment than in a quiet environment. Determining characteristics of a current acoustic scene in step S14, e.g. volume, noise, etc., can thus improve the cognitive prediction performed in steps S6, S8 by correlating the current activity with the sound environment pattern in step S16. In particular, the current activity pattern of the user may thus not only be provided as a time-dependent diary, but also as a diary dependent on the characteristics of the ambient sound.

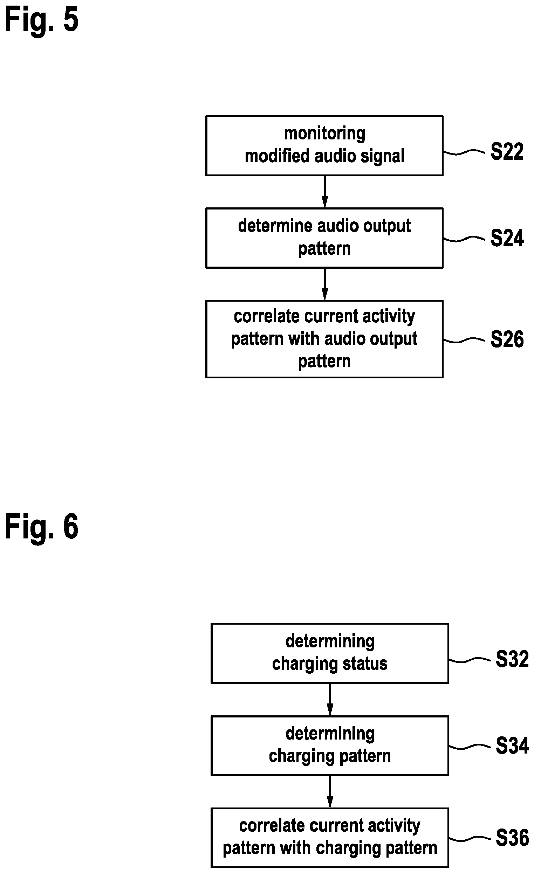

Fig. 5 shows a flow diagram of a method for early detection of a health issue of a user wearing a hearing device, e.g. the above hearing device 12, according to an embodiment of the invention, which may be implemented in conjunction with the method illustrated in Fig. 3 or in conjunction with the methods illustrated in Fig. 3 and Fig. 4. The method may be a computer-implemented method performed automatically in the hearing device 12 and/or the hearing system 60. In step S22, the audio signal modified by the sound processing module 22 is monitored within the predetermined time interval in which the input signal is received in step S2. The monitoring may comprise, for instance, providing information about the modified audio signal, for example by the sound processing control module 26. The information may be received by the health issue determining control module 31. In step S24, an audio output pattern of the hearing device is determined based on the monitoring of the modified audio signal in step S22. The audio output pattern can represent properties of the audio signal outputted to the user at different times. The different times can be associated with corresponding times of the personal parameters and/or social parameters in the current activity pattern of the user. For example, the audio output pattern of the hearing device may comprise parameter values indicative of a distraction level of the user by the audio outputted by the hearing device.

In step S26, the current activity pattern determined in step S4 is correlated with the audio output pattern. The evaluating the current activity pattern in step S6 can include step 26. For example, the current activity pattern may be correlated with the audio output pattern by inputting the audio output pattern in addition to the current activity pattern in a neural network, for instance by using machine learning or deep learning, e.g. algorithm ML2. The neural network may have been trained in advance to differentiate an activity pattern of a healthy person from an activity pattern of an unhealthy people by also taking into account the audio output pattern associated with the respective activity pattern. The can also improve the evaluation of the current activity pattern in step S6. To illustrate, the audio signal outputted to the user can represent another source of distraction for the user which can have a negative impact when performing daily activities, even more so by a person suffering from cognitive decline.

A prediction whether the user is suffering from a cognitive health issue, as performed in steps S6, S8, may be further improved by combining both of the methods illustrated in Figs. 4 and 5 with the method illustrated in Fig. 3. The current activity pattern determined in step S4 may then correlated with the sound environment pattern, according to step S14, and the audio output pattern, according to step S24. The prediction quality may then be particularly enhanced when the hearing device 12 is employed as a hearing aid, in which the audio signal modified by the sound processing module 22 is employed to improve the user's hearing performance which can, under certain circumstances, lead to a suppression of negative side effects on the cognitive capabilities caused by the environmental sound. To illustrate, a hearing aid may compensate for certain environmental sound influences by executing different hearing programs which are intended to make it easier for the user to hear in those different environmental sound situations. For instance, the ambient noise level can be reduced in an acoustic scene identified by the classification module 27 as "Speech in Noise" in order to make it easier for the user to lead a conversation. This can complicate an assessment of the effects of ambient noise on a cognitive performance. If, for example, an ambient noise is faded out by the hearing aid, the cognitive performance can remain the same or even improve. However, by taking into account, in step S6, both the audio output pattern, as determined in step S24, and the sound environment pattern, as determined in step S14, in correlation with the activity pattern determined in step S4, the prediction of a cognitive health issue can be performed with improved accuracy.

Fig. 6 shows a flow diagram of a method for early detection of a health issue of a user wearing a hearing device, e.g. the above hearing device 12, according to an embodiment of the invention, which may be implemented in conjunction with the method illustrated in Fig. 3 or in conjunction with the methods illustrated in Fig. 3 and Fig. 4 and/or Fig. 5. The method may be a computer-implemented method performed automatically in the hearing device 12 and/or the hearing system 60. In step S32, a charging status of a battery included in the hearing device is determined within the predetermined time interval in which the input signal is received in step S2. The charging status may be determined by the power management module 29. The information may be received by the health issue determining control module 31. In step 34, a charging status pattern of the hearing device is determined based on the received information. For instance, the charging status pattern may include parameters indicative of the charging status of the battery over time, for instance whether the battery is recharging or discharging and/or whether the hearing device is disposed inside a charger or currently used or not used when disposed outside the charger. At step 36, which may be performed during step S6, the current activity pattern of the user is correlated with the charging pattern. Such a correlation can yield further information regarding a possible cognitive health issue of the user. To illustrate, a user suffering from a cognitive decline may rather often forget to charge his hearing device and/or that his hearing device is ready for use but still inside the charger.

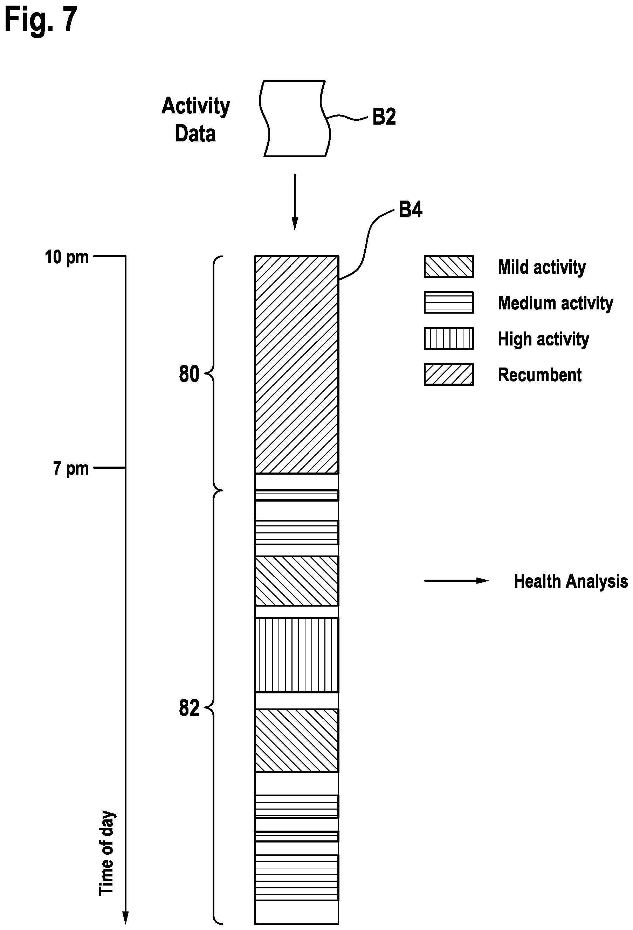

Fig. 7 shows an example of an activity pattern B4, e.g. a daily activity pattern, of a user wearing a hearing device, e.g. the above hearing device 12. The daily activity pattern is generated by evaluating activity data B2, which have been collected for e.g. 24 hours. For example, the audio signal and/or the user detection signal of the hearing device 12 may be monitored for 24 hours in order to collect the activity data B2 and the corresponding data may be stored in the memory 30, 38. The daily activity pattern may be generated from parameter values, which have been extracted and/or derived from the audio signal and/or the user detection signal. For example, depending on this data, e.g. from the sensor 21 being an accelerometer, it may be determined that the user enjoyed a mild activity, a medium activity, or a high activity, or that the user was recumbent.

The daily activity pattern may thus comprise information about the personal parameters, which may include activity parameters and/or physiological parameters and/or dexterity parameters and/or speech parameters of the user, and/or the social parameters. For example, with respect to the physiological parameters, the daily activity pattern may comprise information about the pulse, blood pressure, blood oxygen saturation, and/or body temperature of the user. Further, with respect to the dexterity parameters, the daily activity pattern may comprise information about durations of, speeds of, and/or pauses between single or double taps, taps and hold, and/or swipes up/down of the input mean 28 and/or at the graphical user interface 40. In particular, users having a health issue tend to need longer for manipulating the input mean 28 and/or at the graphical user interface 40, and/or tend to make longer pauses between single manipulations or double taps, and/or tend to manipulate the input mean 28 and/or at the graphical user interface 40 more slowly than a healthy user. Furthermore, with respect to the social parameters, the daily activity pattern may comprise information about a contact of the user to other people, whether, how often, and/or how long the user listens and/or talks to other people, and/or the time the user spent in different social situations. Moreover, the sleeping time of the user and/or the charging time of the hearing device 12 may also give information about the health state of the user and may also be integrated into the activity pattern.

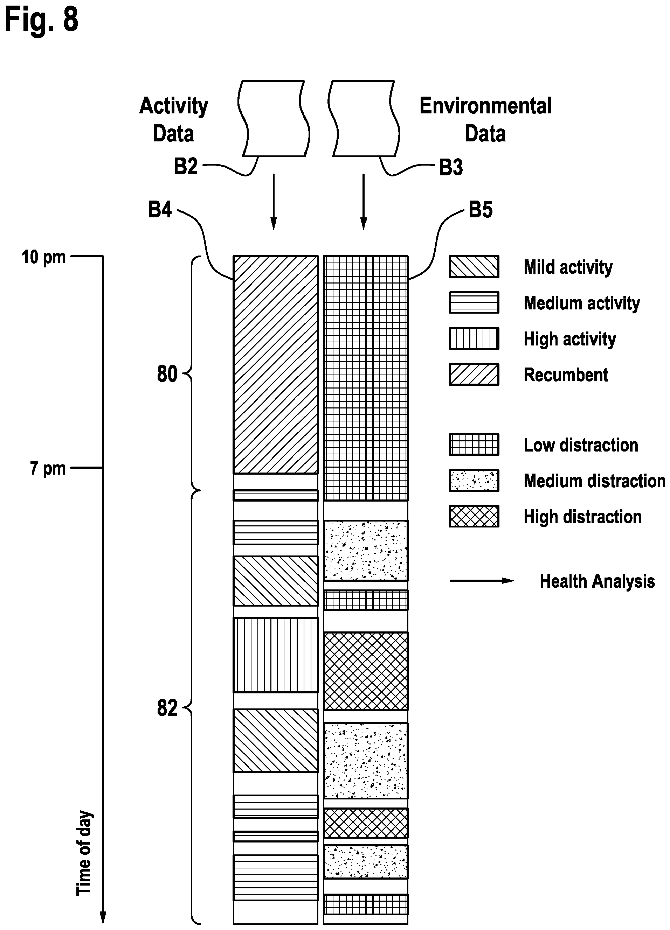

Fig. 8 shows an example of a sound environment pattern B5 that can be correlated with the activity pattern B4. The sound environment pattern is generated by evaluating environmental sound data B3, which also has been collected within the time in which the activity data B2 has been collected. Different times in the activity pattern B4 thus correspond to different times in the sound environment pattern B5. For example, the audio signal of the sound detector 20 may be monitored for 24 hours in order to collect the environmental sound data B3. The audio signal may also be classified by the sound classifier 27, 48 and/or another property the audio signal, such as a signal to noise ratio and/or frequency content and/or volume level, may be determined. The corresponding data may be stored in the memory 30, 38. The sound environment pattern may thus be generated from parameter values, which have been extracted and/or derived from the audio signal. The parameter values of the sound environment pattern can indicate a distraction level of the user which can have an impact on the cognitive performance of the user and can thus be helpful to determine a cognitive impairment. For instance, as illustrated, the parameter values may represent a low distraction, medium distraction or high distraction of the user by the environmental sound.

Similar to the sound environment pattern B5, an audio output pattern of the hearing device 12 may be generated based a monitoring of the audio signal modified by the sound processing module 22. E.g., the modifying of the audio signal may be monitored for 24 hours in order to collect data characteristic for the audio output based on which the audio output pattern may be determined. The corresponding data may be stored in the memory 30, 38. The parameter values of the audio output pattern can also indicate a distraction level of the user influencing his cognitive performance.

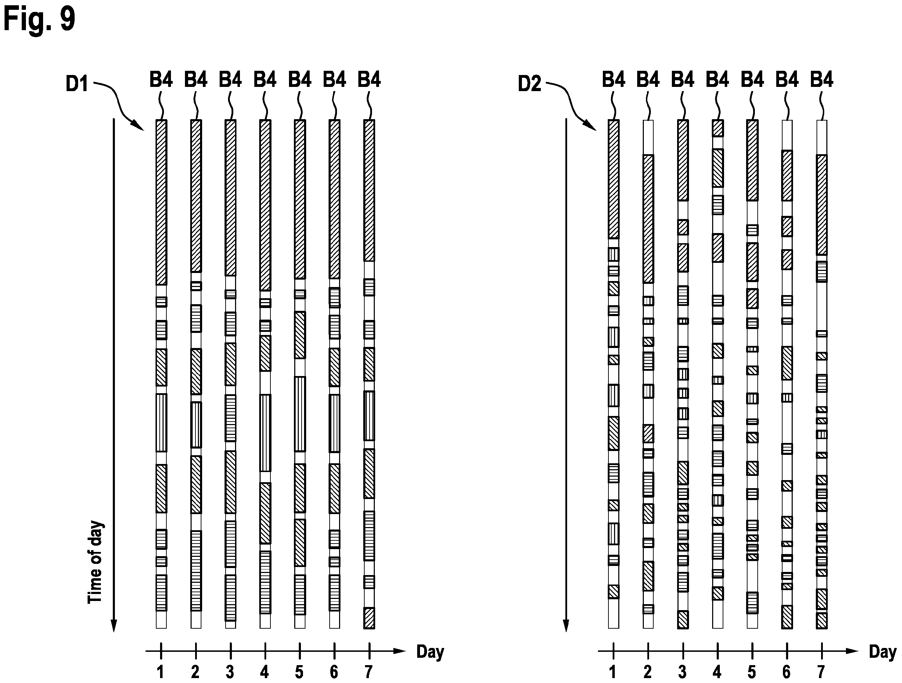

Fig. 9 shows two examples of activity patterns, e.g. long-term activity patterns, e.g. weekly, 20-day, or monthly activity patterns, of a user wearing a hearing device, e.g. the above hearing device 12. In particular, figure 9 shows a first activity pattern D1 of a healthy user and a second activity pattern D2 of an unhealthy user. The activity patterns D1, D2 may comprise information about the sleeping time, the grooming time, get-ready-for-bed time, TV-time, toilet time, cooking time, eating time, mild/seating activity time, visitor time, and out-of-home time, which may be encoded in parameter values of the corresponding parameters. Each column of the activity patterns D1, D2 is representative for a one day activity pattern and/or one 24h-activity pattern of the corresponding user.

It may be seen from figure 9 that the "healthy" first activity pattern D1 clearly differs from the "unhealthy" second activity pattern D2. Therefore, the first activity pattern D1 may be easily differentiated from the second activity pattern D2 by a corresponding algorithm analysing the differences between the first activity pattern D1 and the second activity pattern D2, or by a neural network which has been trained with a large amount of "healthy" first activity patterns D1 and/or "unhealthy" second activity patterns D2.

Further, there may be several classes of "unhealthy" second activity patterns D2, wherein each class is associated with a corresponding health issue. Then, the algorithm or the neural network may be configured and/or trained to differentiate between different health issues.

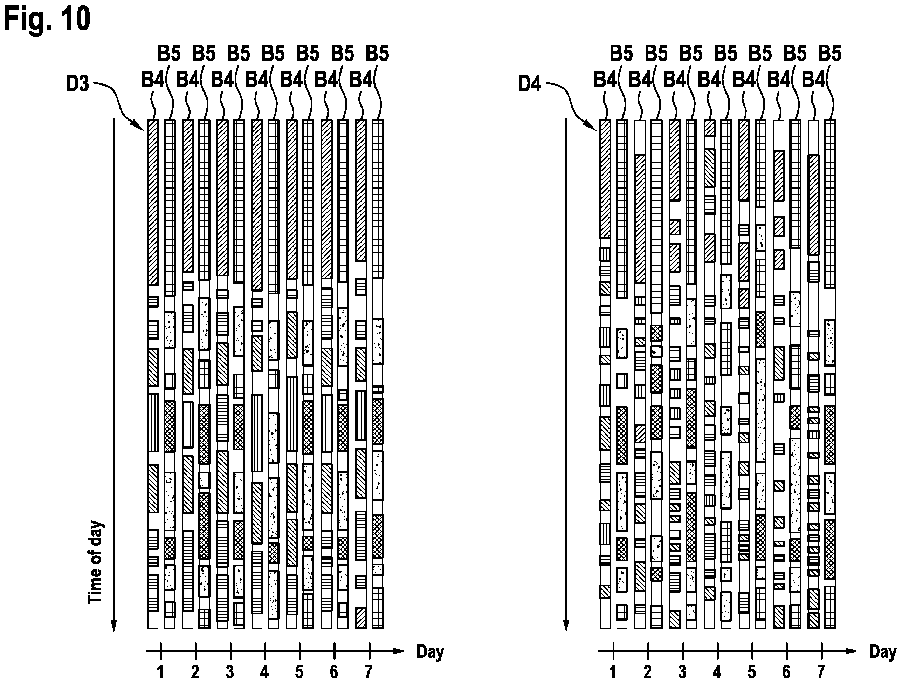

Fig. 10 shows two examples of long-term correlation patterns D3, D4, in which activity patterns B4 are correlated with sound environment patterns B5, e.g. on a weekly or monthly basis. In particular, figure 10 shows a first correlation pattern D3 of a healthy user and a second correlation pattern D4 of an unhealthy user. It may be seen from figure 10 that the "healthy" correlation patterns D3 clearly differs from the "unhealthy" correlation patterns D4. It may further be seen that environmental disturbances, as indicated by the sound environment patterns B5, have a smaller impact on a duration in which high and/or medium activities are carried out in the "healthy" correlation pattern D3 as compared to the "unhealthy" correlation patterns D4.

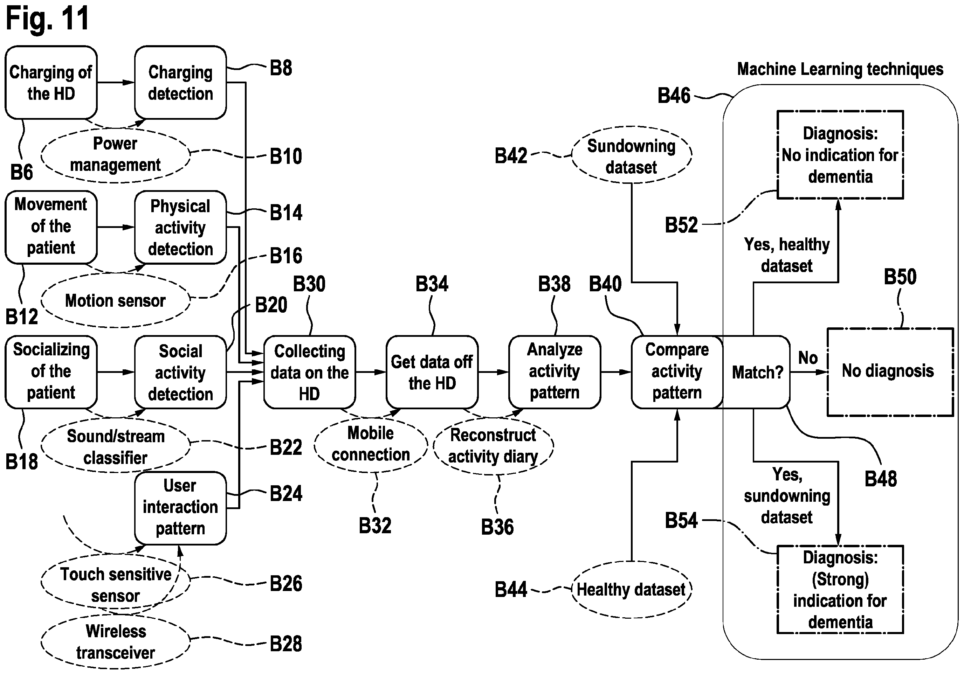

Fig. 11 shows a schematic block diagram of a method for early detection of a health issue of a user wearing a hearing device, e.g. the above hearing device 12, according to an embodiment of the invention. The method visualized by the block diagram may correspond to the method explained above, wherein the block diagram shows more details than the above flow diagram.

Block B6 is representative for the charging of the hearing device 12. Block B8 is representative for the detection of the charging. Block B10 is representative for the power management of the hearing device 12 or the user device 70 detecting the charging of the hearing device 12. The power management may provide the information about the charging and the corresponding data to the processing unit of the hearing device 12 and/or the user device 70.

Block B12 is representative for a movement of the user, e.g. a patient. Block B14 is representative for the detection of the physical activity of the user, e.g. the movement of the user or of a body part of the user, e.g. of the head or of an arm of the user. Block B16 is representative for a motion sensor, e.g. sensor 21, of the hearing device 12. The motion sensor may provide the information about the movement user and the corresponding data to the processing unit of the hearing device 12 and/or the user device 70.

Block B18 is representative for the socializing of the user, i.e. the patient. Block B20 is representative for the detection of the social activity of the user, i.e. the socializing of the user. Block B22 is representative for the sound classifier 27, 48, which may detect, for instance, a social activity of the user. The sound classifier 27, 48 may provide the information about the socializing of the user and the corresponding data to the processing unit of the hearing device 12 and/or the user device 70.

Block B24 is representative for a user interaction pattern of the user. Block B26 is representative for a touch-sensitive sensor, e.g. input mean 28 or control element 44, for detecting the parameter values from which the user interaction may be derived. So, the user interaction pattern may include interaction activities on the hearing device 12 and/or the graphical user interface 40 of the user device 70, which may be transferred from the hearing device 12 to the graphical user interface 40 and/or vice versa to obtain a full picture of the interactions of the user. From this information, dexterity parameters of the user may be derived. Block B28 is representative for a wireless transceiver, e.g. the first and/or the second transceiver 32, 34, for monitoring the mobile data connections of the hearing device, which may be used to derive the user interaction pattern. The touch-sensitive sensor and/or the wireless transceiver may send the information for deriving the user interaction pattern and the corresponding data to the controller of the hearing device 12 or the user device 70.

Block B30 is representative for collecting the data or at least a part of the data in the hearing device 12, e.g. in the first memory 30. At least parts of the data may originate from sensors of the connected user device 70 and/or of one or more further user devices worn by the user, e.g. on the body of the user, e.g. a smartphone, smartwatch, fitbit, etc. The data may be the raw data (e.g. from a sensor) or may be pre-processed (e.g. cleansed, reduced, enriched, re-sampled, filtered) upon collection. Block B32 is representative for a mobile connection of the hearing device 12 to the user device 70, which may be used to transfer the data stored in the hearing device 12 to the user device 70, e.g. to the second memory 38. Block B34 is representative for the data being received by the connected user device 70. Upon reception of the data, further pre-processing for data preparation may be conducted on the user device 70.

Block B36 is representative for determining the current activity patterns by recognizing the single activities from the data and reconstructing and/or generating an activity dairy, e.g. the current activity pattern, which may be a daily activity pattern, of the user. Alternatively, the activity dairy may be a weekly, monthly, or multiple months activity pattern. The activity recognition may rely on signal processing methods or may use machine learning methods (e.g. Hidden Markov Models or Conditional Random Fields) or deep learning methods (e.g., Convolutional Neural Networks, Recurrent Neural Networks like Long Short-Term Memory networks, or a combination thereof, such as deep convolutional LSTM networks). The data may be organized in sequences or windows of certain size and duration (e.g. 1 second of data). Each such sequence or window is associated with a specific activity, such as sleeping or walking, and can serve as an input to a deep neural network for training and inference. Block B38 and block B40 are representative for evaluating the current activity patterns. Block B38 is representative for analysing the current activity pattern, e.g. creating activity maps or images, or extracting specific features from an activity pattern. Block B40 is representative for comparing the current activity pattern with a "healthy" activity pattern, e.g. a "healthy" dataset B44, and/or with an "unhealthy" activity pattern, e.g. a sundowning dataset, e.g. by means of map or feature matching, or classification techniques. Block B40 may be integrated in Block 38, such that the comparison is carried out when analysing the corresponding data. The term "comparing" may be interpreted in a very broad way such that it may refer to comparing the parameter values of the current activity pattern with the parameter values of the previous activity pattern, i.e. the "healthy" dataset and/or the "unhealthy" dataset, e.g. by a rule-based, tree-based, or probabilistic algorithm, and/or that it may refer to a neural network, which has been trained with the "healthy" dataset and/or the "unhealthy" dataset in advance and which is able to differentiate between the "healthy" dataset and/or the "unhealthy" dataset. For example, the classification into one or multiple "healthy" and "unhealthy" classes, possibly each of which characteristic of a health issue or disease pattern, can be realized by deep neural networks, which make use of one or several hidden layers.

As seen from above, the activity recognition step in Block B36 as well as the comparison step in Block B40 may both be formulated as classification problems, which can be addressed by supervised learning. The used models (e.g. of a deep neural network) are trained (e.g. for setting the weights and bias of the network), validated (e.g. for identifying optimal hyperparameters of the network, such as a reasonable number of hidden layers) and tested (e.g. for evaluating and benchmarking the network's performance) with datasets, which are at least in part labelled. The datasets can be proprietary (e.g. from our company-owned real-life media data bases) or publicly available (e.g. benchmark datasets like mHealth, Bern Cocktail Party, Opportunity, or others).Block B48 is representative for a determination whether there is an upcoming health issue of the user or not. For example, in a classification scenario, the determination may be as simple as selecting the most likely class resulting as output from the comparison in Block B40. Block B50 is representative for the decision that no diagnosis may be done, i.e. that the diagnosis is not possible. Block B52 is representative for the diagnosis that there is no health issue, e.g. that there is no indication for dementia. Block B54 is representative for the diagnosis that there is at least a strong indication or already is a health issue, e.g. that there is a strong indication for dementia. Alternatively, other brain related health issues may be diagnosed with the above method, as explained above. Further, there may be more or less parameter values and corresponding activities and/or behaviours detected during carrying out the method, wherein the current activity pattern may be generated based on these parameter values.

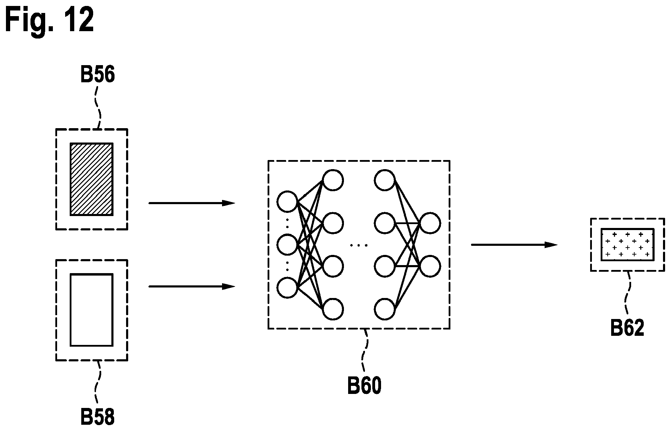

Fig. 12 shows a schematic block diagram visualising at least in part an exemplary embodiment of the method for early detection of the health issue of the user wearing the hearing device 12, according to an embodiment of the invention, which may be implemented in conjunction with any of the methods illustrated in figures 3 to 6 and 11.

Block B56 represents a data set comprising at least one audio parameter 122 (see figure 12). The audio parameter 122 represents at least in part the current acoustic environment of the user and/or information regarding the modified audio signal modified by the sound processing module 22 of the hearing device 12. The audio parameter 122 may thus be represented by the sound environment pattern and/or the audio output pattern described above. For example, the audio parameter values of one of the audio parameters 122 representing the current acoustic environment may be derived directly from the received audio signal, e.g. an acoustic level and/or frequency spectrum of the audio signal, or indirectly from the received audio signal, e.g. a classification value generated by one of the classifiers 27, 48 of the hearing system 60 depending on the received audio signal or a differential value characterizing a difference between the received audio signal and the modified audio signal. In contrast, the audio parameters 122 representing the information regarding the modified audio signal may be derived from the modified audio signal itself, e.g. an acoustic level, content, and/or frequency spectrum of the modified audio signal.

Block B58 represents the above current activity pattern determined by evaluating the input signal.

Block B60 represents the neural network which determines whether there is the upcoming health issue or not depending on the at least one audio parameter received from block B56 and on the current activity pattern received from block B58.

So, according to the method of figure 7, the upcoming health issue may be determined depending not only on the current activity pattern, but also depending on the audio parameter 122.

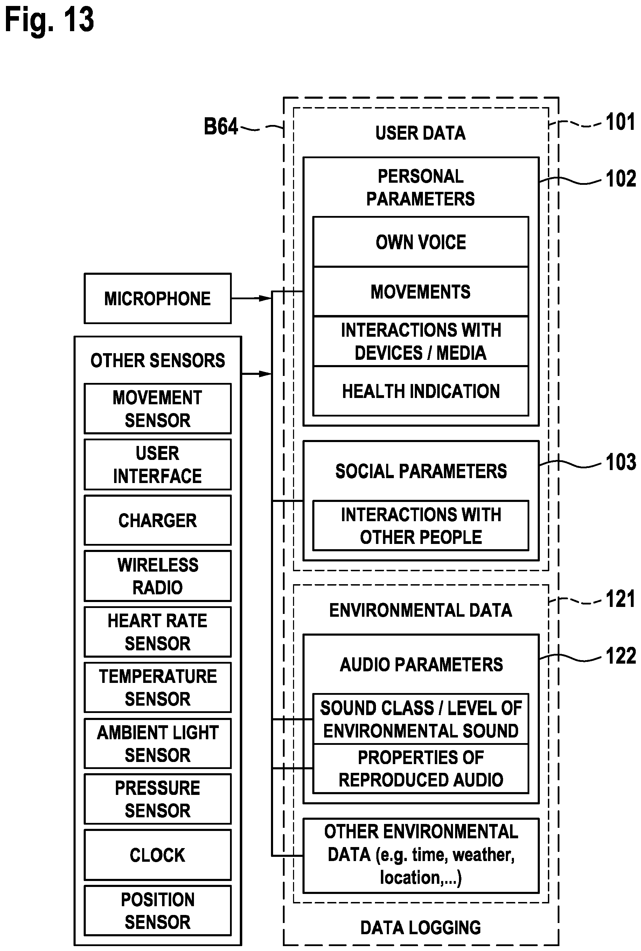

Fig. 13 shows a schematic block diagram visualising exemplary embodiments of sensors and parameters usable for carrying out the method for early detection of the health issue of the user wearing the hearing device 12, according to an embodiment of the invention. In particular, figure 12 visualizes the parameters, which may be logged in order to generate one or more activity patterns and/or additional information that can be correlated with the activity patterns, such as sound environment patterns and/or audio output patterns and/or charging status patterns, in order to determine whether there is the upcoming health issue, and the corresponding sensors. In particular, block 64 represents all the data which may be logged. This data may be user data 101 and environmental data 121.

The user data 101 may be used for generating the current activity pattern. The user data may comprise personal parameters, such as own voice of the user, movements of the user, interactions of the user with devices/media, and/or a health indication of the user. The environmental data 121 may be used for determining whether there is the upcoming health issue or not. In particular, the environmental data 121 may correspond to the data set of audio parameters which are visualized by block B56 of figure 11. For example, the environmental data 121 comprise the above audio parameters 122, e.g. the current sound class, the level of environmental sound, and/or properties of the modified audio signal.

Optionally, the environmental data also includes other environmental parameters and their corresponding parameter values, e.g. a current time, weather, location of the user, etc. These environmental data may be additionally used for determining whether there is the upcoming health issue or not, e.g. as another input block for the neural network represented by block B60 in figure 11, or as further parameters within the data set of block B56 of figure 11.

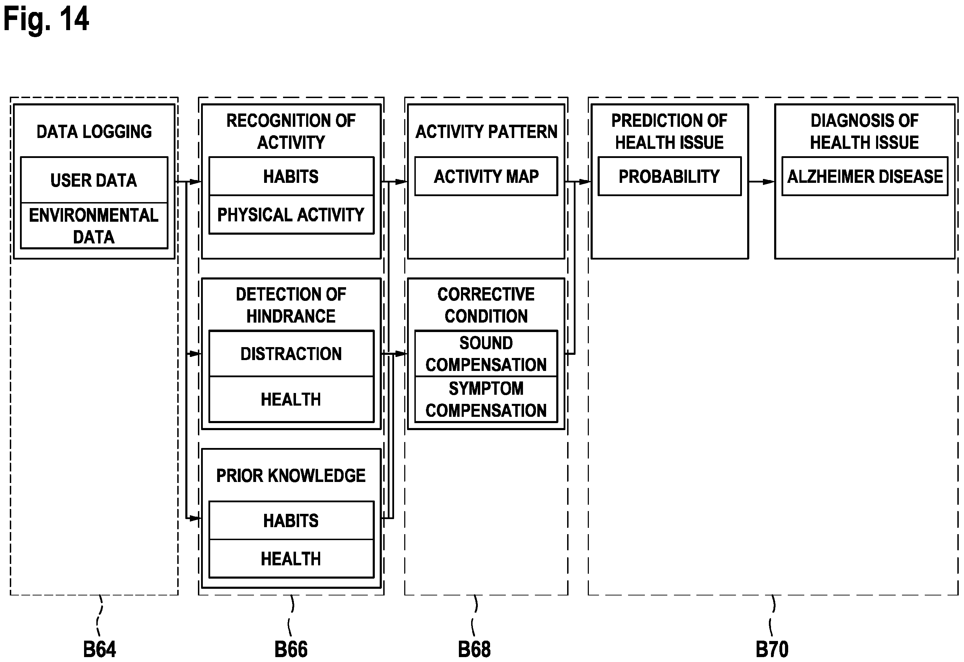

Fig. 14 shows a schematic block diagram visualising at least in part an exemplary embodiment of a method for early detection of the health issue of the user wearing the hearing device 12, which may be implemented in conjunction with any of the methods illustrated in figures 3 to 6, 11, and 12.

Block B64 is representative for a data logging. The logging of the data may be done e.g. by continuously measuring and reading the data by the sound detector and/or user detector of the hearing device 12 and/or hearing system 60 and/or, at least in part, by inputting additional data during a fitting process for fitting the hearing device 12 to the user, which may be carried out by a health care professional (HCP).

Block B66 is representative for the recognition of the current activity of the user, e.g. his/her habits and/or physical activity, for the detection of hindrance, e.g. a distraction of the user and/or a health of the user, and/or for prior knowledge, e.g. about the habits and/or the health of the user. For example, the detection of the hindrance may be determined with the help of at least one of the classifiers, e.g. in order to calculate a sound compensation, e.g. of noise, e.g. by adjusting the weights of the neural network, which may be considered at the inference of the health issue diagnosis. Block B66 may be carried out at least in part by machine learning or deep learning approaches, e.g. ML1 and ML2.

Block B68 is representative for the determined current activity pattern, in other words an activity map, and/or a corrective condition, e.g. a sound compensation in accordance with one of the sound programs and/or a symptom compensation of the user.

Block B70 is representative for the prediction of the upcoming health issue, in particular its probability, and optionally for the diagnosis of the health issue, e.g. Alzheimer disease. Block B70 may be carried out at least in part by machine learning or deep learning approaches, e.g. ML1 and ML2. Benchmarks and comparisons via similarity measures, e.g. correlation, mutual information, etc., may be used within block B70, e.g. healthy activity patterns, unhealthy activity patterns, e.g. sundowning activity patterns. Further, the final decisions regarding the presence or prediction of a particular diagnosis may be generated from the above probabilities. For example, the final diagnosis is only done, if the calculated probabilities, e.g. as average value, indicates the diagnosis over a predetermined amount of time.

As visualised by the arrows extending from one block of figure 13 to another block of figure 13, the blocks B66 to B70 use the results and values presented by the corresponding previous block in order to produce the corresponding output, which is forwarded from blocks B66 to B68 to the corresponding next block.

Block B64, i.e. generating and receiving the corresponding parameter values, may be carried out by the hearing device 12 and optionally at least in part by the connected user device 70. Blocks B66 to B70 may be carried out by the user device 70, another device worn by the user, and/or a cloud server of the internet. Alternatively, blocks B64 and B66 may be carried out by the hearing device 12 and optionally at least in part by the connected user device 70, and blocks B68 and B70 may be carried out by the user device 70, another device worn by the user, and/or a cloud server of the internet. Alternatively, blocks B64 to B68 may be carried out by the hearing device 12 and optionally at least in part by the connected user device 70 and block B70 may be carried out by the user device 70, another device worn by the user, and/or a cloud server of the internet.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art and practicing the claimed invention, from a study of the drawings, the disclosure, and the appended claims. In the claims, the word "comprising" does not exclude other elements or steps, and the indefinite article "a" or "an" does not exclude a plurality. A single processor or controller or other unit may fulfil the functions of several items recited in the claims. The mere fact that certain measures are recited in mutually different dependent claims does not indicate that a combination of these measures cannot be used to advantage. Any reference signs in the claims should not be construed as limiting the scope.

### LIST OF REFERENCE SYMBOLS

- 12: hearing device
- 15: part behind the ear
- 16: part in the ear
- 18: tube
- 20: sound detector
- 21: sensor
- 22: sound processing module
- 23: processing unit
- 24: sound output component
- 26: sound processing control module
- 27: sound evaluation module
- 28: input mean
- 29: power manager
- 30: memory
- 31: health issue determining control module
- 32: transceiver
- 34: transceiver
- 36: processing unit
- 38: memory
- 40: graphical user interface
- 42: display
- 44: control element, slider
- 48: classifier
- 60: hearing system
- 70: connected user device
- 80: charging time
- 82: no-charging time
- 101: user data
- 102: personal parameters
- 103: social parameters

- 121: environmental data
- 122: audio parameters
- S2 to S36: steps two to thirty six
- B2 to B70: blocks two to seventy
- D1, D2: activity patterns
- D3, D4: activity patterns correlated with sound environment patterns

## Claims

1. A method for early detection of a health issue of a user of a hearing device (12) configured to be worn at an ear of the user, the hearing device (12) comprising at least one sound detector (20) for generating an audio signal indicative of a sound detected in an environment of the user, at least one user detector (21, 27, 29) for generating a user detection signal indicative of a property of the user and/or an activity performed by the user, a processing unit (23, 36) for modifying the audio signal, and at least one sound output component (24) for outputting the modified audio signal, the method comprising:
receiving (S2) the audio signal and/or the user detection signal for a predetermined time interval;
determining (S4) a current activity pattern of the user by evaluating the audio signal and/or the user detection signal received over the predetermined time interval;
evaluating (S6) the current activity pattern;
determining (S8) whether there is an upcoming health issue of the user depending on the evaluation; and
providing (S10), if it is determined that there is an upcoming health issue of the user, an output signal indicative of the health issue.

2. The method of claim 1, wherein
the current activity pattern is evaluated by a neuronal network, which has been trained in advance to differentiate an activity pattern (D1) of a healthy person from an activity pattern (D2) of an unhealthy person.

3. The method of claim 1, wherein
the current activity pattern is evaluated by comparing the current activity pattern with at least one previous activity pattern of the user, and
it is determined that there is an upcoming health issue of the user if the current activity pattern differs from the previous activity pattern in a predetermined way.

4. The method of claim 3, wherein
the previous activity pattern represents an average of previously determined and stored activity patterns of the user.

5. The method of one of claims 3 or 4, wherein
after evaluating the current activity pattern, the previous activity pattern is adapted depending on the current activity pattern.

6. The method of one of the preceding claims, wherein evaluating the current activity pattern comprises:
determining which health issue comes up based on previous activity patterns of unhealthy people having the corresponding health issue, and
providing the predetermined early detection signal indicating the upcoming health issue, if it is determined that there is an upcoming health issue of the user.

7. The method of one of the previous claims, wherein
the health issue refers to the health of the brain of the user.

8. The method of one of the preceding claims, wherein
the activity pattern of the user comprises parameter values of a set of parameters, wherein the parameters comprise at least one of the group of
- activity parameters indicative of a physical and/or mental activity performed by the user;
- physiological parameters indicative of a physiological property of the user;
- dexterity parameters indicative of a manual dexterity of the user;
- speech parameters indicative of a speaking capability of the user;
- social parameters indicative of a contact of the user to other people, whether, how often, and/or how long the user listens and/or talks to other people, and/or a number of people the user interacts with, and/or a frequency in which the user interacts with different people, and/or a time the user spent in different social situations, like quiet situations and/or situations involving other people speaking.

9. The method of one of the preceding claims, further comprising:
determining, based on the received audio signal generated by the sound detector (20), a sound environment pattern of the user,
wherein said evaluating (S6) the current activity pattern comprises correlating the current activity pattern of the user with the sound environment pattern.

10. The method of claim 9, further comprising:
classifying the received audio signal generated by the sound detector (20) by assigning the audio signal to a class from a plurality of predetermined classes, wherein the sound environment pattern of the user is determined based on the class assigned to the audio signal; and/or
determining a volume level of the received audio signal generated by the sound detector (20), wherein the sound environment pattern of the user is determined based on the determined volume level.

11. The method of one of the preceding claims, further comprising:
monitoring the modifying of the audio signal within the predetermined time interval;
determining, based on the monitored modifying of the audio signal, an audio output pattern of the hearing device,
wherein said evaluating (S6) the current activity pattern comprises correlating the current activity pattern of the user with the audio output pattern.

12. The method of one of the preceding claims, further comprising:
determining a charging status of a battery included in the hearing device;
determining, based on the charging status, a charging status pattern of the hearing device,
wherein said evaluating (S6) the current activity pattern comprises correlating the current activity pattern of the user with the charging pattern.

13. A hearing device (12) configured to be worn at an ear of a user, the hearing device comprising
at least one sound detector (20) for generating an audio signal indicative of a sound detected in an environment of the user;
at least one user detector (21, 27, 29) for generating a user detection signal indicative of a property of the user and/or an activity performed by the user;
a processing unit (23) for modifying the audio signal; and
at least one sound output component (24) for outputting the modified audio signal,
wherein the processing unit (23) is configured to carry out the method in accordance with one of the preceding claims.

14. The hearing device (12) of claim 13, wherein the user detector comprises
- an own voice detector configured to detect an own voice activity of the user;
and/or
- a sound classifier configured to identify a sound class in the audio signal generated by the sound detector; and/or
- a movement sensor configured to detect a movement of the hearing device;
and/or
- a biometric sensor configured to detect a biometric property of the user; and/or
- a user-interface configured to detect a user interaction; and/or
- a location detector configured to detect a location of the user and/or a change of location of the user; and/or
- a power manager configured to detect a charging status of a battery associated with the hearing device.

15. A hearing system (10) comprising a hearing device (12) configured to be worn at an ear of a user and a user device (70) connectable to the hearing device (12) for data exchange with the hearing device (12), the hearing device comprising
at least one sound detector (20) for generating an audio signal indicative of a sound detected in an environment of the user;
a processing unit (23, 36) for modifying the audio signal; and
at least one sound output component (24) for outputting the modified audio signal; the hearing system further comprising
at least one user detector (21, 27, 29) for generating a user detection signal indicative of a property of the user and/or an activity performed by the user,
wherein the processing unit (23, 36) is configured to carry out the method in accordance with one of claims 1 to 12.

16. A computer program for early detection of a health issue of a user wearing a hearing device (12), the computer program, when being executed by a processing unit (23, 36), being adapted to carry out the steps of the method of one of claims 1 to 12.
